(19) **Europäisches Patentamt European Patent Office Office européen des brevets**

(11) **EP 4 763 968 A1**

(12) **EUROPEAN PATENT APPLICATION**
published in accordance with Art. 153(4) EPC

(43) Date of publication:
**24.06.2026 Bulletin 2026/26**

(21) Application number: **24854079.1**

(22) Date of filing: **28.06.2024**

(51) International Patent Classification (IPC):
**C12M 1/34** (2006.01)  **C12M 1/00** (2006.01)
**C12Q 1/02** (2006.01)  **G02B 21/00** (2006.01)

(52) Cooperative Patent Classification (CPC):
**C12M 1/00; C12M 1/34; C12Q 1/02; G02B 21/00**

(86) International application number:
**PCT/JP2024/023605**

(87) International publication number:
**WO 2025/037488 (20.02.2025 Gazette 2025/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.08.2023 JP 2023132533**

(71) Applicant: **NIKON CORPORATION Tokyo 140-8601 (JP)**

(72) Inventors:
• **HAYASHI, Kohma Tokyo 140-8601 (JP)**

• **HOSHINO, Tetsuro Tokyo 140-8601 (JP)**
• **ABE, Takeyuki Tokyo 140-8601 (JP)**
• **OI, Hiromi Tokyo 140-8601 (JP)**
• **IWAMOTO, Chisako Tokyo 140-8601 (JP)**
• **ONISHI, Shutaro Tokyo 140-8601 (JP)**

(74) Representative: **Hoffmann Eitle Patent- und Rechtsanwälte PartmbB Arabellastraße 30 81925 München (DE)**

(54) **OBSERVATION DEVICE, OBSERVATION METHOD, AND PROGRAM**

(57) An observation device that analyzes a cell image includes an image acquiring unit configured to acquire a second image in a region wider than a region used for analysis at a pixel resolution lower than a pixel resolution of a first image used for the analysis, an index calculating unit configured to calculate a value of an index for a cell in each of subregions included in the second image and corresponding to the region used for the analysis, and a region selecting unit configured to select a region to be used for the analysis from the subregions on the basis of the value of the index calculated by the index calculating unit.

FIG. 1

EP 4 763 968 A1

**Description**

TECHNICAL FIELD

The present invention relates to an observation device, an observation method, and a program.
Priority is claimed on Japanese Patent Application No. 2023-132533, filed August 16, 2023, the content of which is incorporated herein by reference.

BACKGROUND ART

In the biological sciences, the medical sciences, and the like, it is known that a condition such as health or disease of an organism is associated with, for example, conditions of cells or cell organelles. Accordingly, analysis of this association serves as a means for solving various problems in the biological sciences, the medical sciences, and the like. Analysis of an intercellular or intracellular information transmission path can be usefully used for, for example, studies of industrial biosensors or pharmaceuticals for disease prevention. For example, a technique using image processing is known as one analysis technique for cells, tissue slices, or the like (for example, see Patent Document 1).

Citation list

Patent Document

Patent Document 1: United States Patent No. 9280693

SUMMARY OF INVENTION

According to an aspect of the present invention, there is provided an observation device that analyzes a cell image, the observation device including an image acquiring unit configured to acquire a second image in a region wider than a region used for analysis at a pixel resolution lower than a pixel resolution of a first image used for the analysis, an index calculating unit configured to calculate a value of an index for a cell in each of subregions included in the second image and corresponding to the region used for the analysis, and a region selecting unit configured to select a region to be used for the analysis from the subregions on the basis of the value of the index calculated by the index calculating unit.
According to another aspect of the present invention, there is provided an observation method of analyzing a cell image, the observation method including acquiring a second image in a region wider than a region used for analysis at a pixel resolution lower than a pixel resolution of a first image used for the analysis, calculating a value of an index for a cell in each of subregions included in the second image and corresponding to the region used for the analysis, and selecting a region to be used for the analysis from the subregions on the basis of the value of the index calculated in the calculating of a value of an index.
According to another aspect of the present invention, there is provided a program causing a computer that analyzes a cell image to perform acquiring a second image in a region wider than a region used for analysis at a pixel resolution lower than a pixel resolution of a first image used for the analysis, calculating a value of an index for a cell in each of subregions included in the second image and corresponding to the region used for the analysis, and selecting a region to be used for the analysis from the subregions on the basis of the value of the index calculated in the calculating of a value of an index.

BRIEF DESCRIPTION OF DRAWINGS

[FIG. 1] A diagram illustrating an example of a configuration of an observation device according to a first embodiment.
[FIG. 2] A block diagram illustrating an example of a functional configuration of the observation device according to the first embodiment.
[FIG. 3] A diagram illustrating an example of a configuration of a microscope device according to the first embodiment.
[FIG. 4] A diagram illustrating an example of index types according to the first embodiment.
[FIG. 5] A diagram illustrating an example of combinations of a first index and a second index according to the first embodiment.
[FIG. 6] A diagram illustrating an example of a flow of a first preparation process according to the first embodiment.
[FIG. 7] A diagram illustrating an example of a flow of an observation region selecting process according to the first embodiment.

[FIG. 8] A diagram illustrating an example of a pre-image according to the first embodiment.

[FIG. 9] A diagram illustrating an example of a flow of a second preparation process according to the first embodiment.

[FIG. 10] A diagram illustrating an example of a flow of a main image capturing and analyzing process according to the first embodiment.

[FIG. 11] A block diagram illustrating an example of a functional configuration of an observation device according to a second embodiment.

[FIG. 12] A diagram illustrating an example of a flow of an observation region selecting process according to the second embodiment.

[FIG. 13] A block diagram illustrating an example of a functional configuration of an observation device according to a third embodiment.

[FIG. 14] A diagram illustrating an example of a flow of a first preparation process according to the third embodiment.

[FIG. 15] A diagram illustrating an example of a pre-image in which foreign matter is imaged according to the third embodiment.

[FIG. 16] A diagram illustrating an example of a flow of an observation region selecting process according to the third embodiment.

DESCRIPTION OF EMBODIMENTS

Hereinafter, the present invention will be described in conjunction with embodiments of the invention. The following embodiments do not limit the inventions described in the appended claims. All combinations of features described in the embodiment cannot be said to be essential for the solution of the present invention.

(First embodiment)

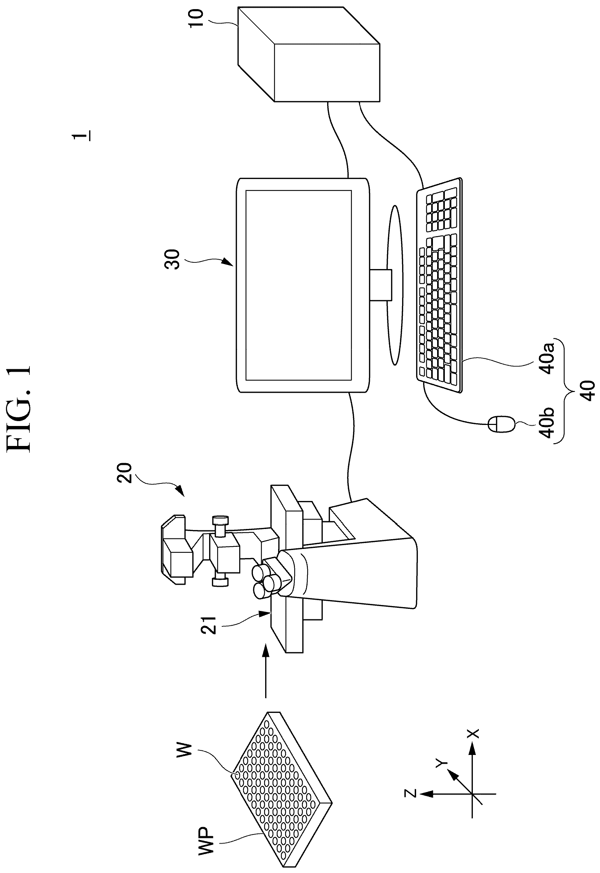

A first embodiment will be described below in detail with reference to the accompanying drawings. In the following description, an XYZ orthogonal coordinate system is set, and positional relationships of constituents will be described with reference to the XYZ orthogonal coordinate system. A plane perpendicular to an optical axis of an objective lens is defined as an XY plane. A direction parallel to the optical axis of the objective lens is defined as a Z-axis direction. FIG. 1 is a diagram illustrating an example of a configuration of an observation device 1 according to the present embodiment. The observation device 1 is also referred to as an observation system.

The observation device 1 performs image processing on an image in which cells or the like are imaged. The observation device 1 includes a microscope device 20, an analysis device 10, a display unit 30, and an operation unit 40.

The microscope device 20 is a microscope for observing an enlarged image of a sample placed on a motor-driven stage 21. A sample is specifically a biological sample or a bead to be observed. A biological sample is a fluorescence-stained cell with a thickness, or the like. In the following description, an image acquired by imaging a cell or the like is also simply referred to as a cell image.

A well plate WP is held by an exclusive holder and is placed on the motor-driven stage 21. The well plate WP includes one or two or more wells W. In the present embodiment, the well plate WP includes 96 wells W of $8 \times 12$ as illustrated in FIG. 1. The number of wells of the well plate WP is not limited thereto, and the well plate WP may include 6 wells W of $2 \times 3$, 12 wells W of $3 \times 4$, 24 wells W of $4 \times 6$, 384 wells W of $16 \times 24$, 1536 wells W of $32 \times 48$, or 6144 wells W of $64 \times 96$. A cell is cultured in a well W under specific experimental conditions, The specific experimental conditions include a temperature, a humidity, a culturing period, an elapsed time after a stimulus has been given thereto, a type or strength of a stimulus to be given, a concentration, a quantity, whether there is a stimulus, and induction of biological properties. The stimulus is, for example, a physical stimulus such as electricity, sound waves, magnetism, or light or a chemical stimulus based on administration of a matter or a medicine. The biological properties are properties indicating stages or shapes of cell differentiation, the number of cells, behavior of intracellular molecules, shapes or behavior of organelles, forms, behavior of intranuclear bodies, behavior of DNA molecules, behavior and modification of RNA molecules, and behavior and modification of protein molecules. In the following description, the biological properties are also referred to as constituents of a cell. In this example, the microscope device 20 observes cells seeded in a plurality of wells W of the well plate WP. The present invention is not limited to the well plate WP, and a dish (for example, a dish of 35 mm) may be used. In this case, a plurality of dishes are held by an exclusive holder and are placed on the motor-driven stage 21.

The analysis device 10 analyzes an image captured by the microscope device 20. The analysis device 10 is a computer device. In this example, the analysis device 10 determines an observation region which is appropriate for analysis on the basis of a pre-image captured by the microscope device 20. The pre-image is an image which is used to determine an observation region appropriate for analysis. The pre-image is, for example, an image acquired at a magnification lower than a magnification at which a main image is acquired. The main image is an image to be used for image analysis.

In the pre-image, although it depends on a type of the well plate WP, an almost overall view of each well W is imaged at a magnification lower than an imaging magnification of a main image. In the pre-image, only the vicinity of the center of each well W instead of the almost overall view of each well W may be imaged according to a type of the well plate WP.

The analysis device 10 analyzes a main image captured in an observation region.

The display unit 30 displays an image which is analyzed by the analysis device 10, or the like. The image displayed on the display unit 30 includes an image which is generated on the basis of an analysis result from the analysis device 10. The display unit 30 includes, for example, a liquid crystal display.

The operation unit 40 is operated by a user. The operation unit 40 outputs an operation signal when the operation unit 40 is operated by a user. The operation signal is supplied to the analysis device 10. The analysis device 10 acquires various types of information supplied from the user on the basis of the operation signal supplied from the operation unit 40. The operation unit 40 includes, for example, a keyboard 40a and a mouse 40b.

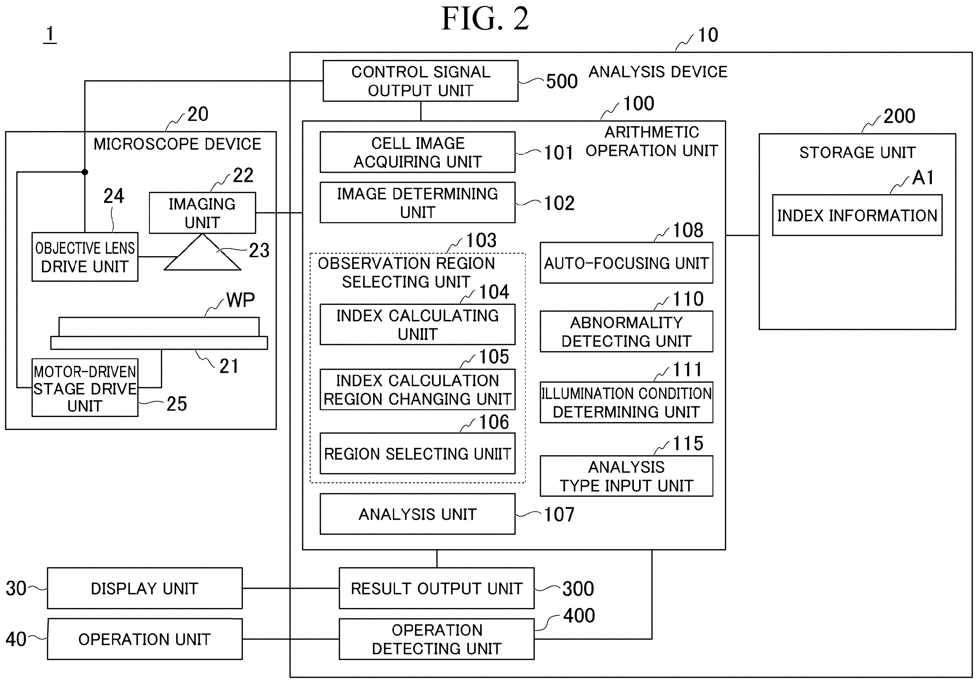

An example of a functional configuration of the observation device 1 will be described below with reference to FIG. 2.

FIG. 2 is a block diagram illustrating an example of the functional configuration of the observation device 1. The microscope device 20 is a biological microscope and includes a motor-driven stage drive unit 25, an imaging unit 22, an objective lens 23, and an objective lens drive unit 24 in addition to the motor-driven stage 21.

The motor-driven stage 21 can arbitrarily move a position of an object in a predetermined direction (for example, any direction in a two-dimensional plane in the horizontal direction). The motor-driven stage 21 is driven by the motor-driven stage drive unit 25. The motor-driven stage drive unit 25 includes a motor that moves the position of the motor-driven stage 21 in an X-axis direction or a Y-axis direction which is a direction perpendicular to the optical axis of the objective lens 23. The motor-driven stage drive unit 25 can move the motor-driven stage 21 in the X-axis direction or the Y-axis direction by driving the motor.

The imaging unit 22 includes an imaging device such as a charge-coupled device (CCD) or a complementary MOS (CMOS) and images an object on the motor-driven stage 21. An image of a cell placed on the motor-driven stage 21 is focused on an imaging surface of the imaging unit 22 by a focusing lens which is not illustrated via the objective lens 23.

The objective lens 23 receives light transmitted by a cell. The light received by the objective lens 23 is not limited to light transmitted by a cell. The objective lens 23 may receive light reflected by a cell. The objective lens 23 may be, for example, a liquid-immersion objective lens such as an oil-immersion objective lens or a water-immersion objective lens or may be a dry objective lens not corresponding to liquid immersion.

The objective lens drive unit 24 includes a motor that moves the position of the objective lens 23 in the Z-axis direction parallel to the optical axis and can move the objective lens 23 in the Z-axis direction by moving a nosepiece (not illustrated) holding the objective lens 23 upward or downward.

More specifically, the microscope device 20 has, for example, a function of a differential interference contrast microscope (DIC), a phase-contrast microscope, a fluorescent microscope, a confocal microscope, a super-resolution microscope, a two-photon excitation fluorescent microscope, a light sheet microscope, a bright-field microscope, a quantitative phase-contrast microscope, or a holographic microscope.

The microscope device 20 images cells in a container placed on the motor-driven stage 21. The container is, for example, a well plate WP, a dish, or a slide chamber. The microscope device 20 captures an image of cells by irradiating cells in a plurality of wells W provided in the well plate WP with light and detecting light transmitted by the cells. Accordingly, the microscope device 20 can acquire a transmissive DIC image, a phase-contrast image, a dark-field image, or a bright-field image of cells.

The microscope device 20 captures an image of cells by irradiating cells stained with fluorescent matter with excitation light and detecting fluorescence generated from the cells.

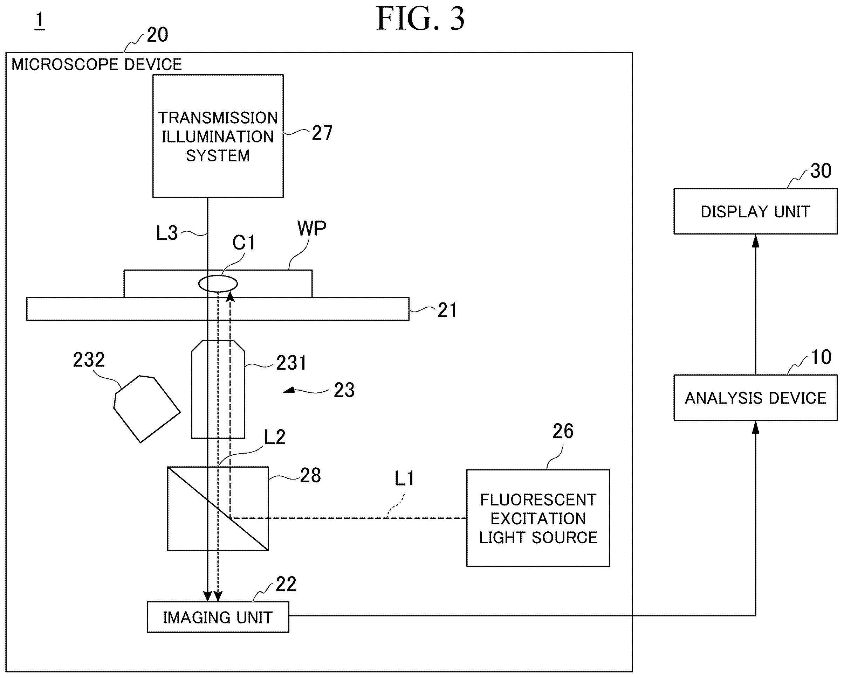

A detailed configuration of the microscope device 20 will be described below with reference to FIG. 3. FIG. 3 is a diagram illustrating an example of a configuration of the microscope device 20 according to the present embodiment. The microscope device 20 includes a fluorescent excitation light source 26 and a transmission illumination system 27 in addition to the motor-driven stage 21, the imaging unit 22, the objective lens 23, the objective lens drive unit 24 (not illustrated in FIG. 2), and the motor-driven stage drive unit 25 (not illustrated in FIG. 2). The objective lens 23 includes a high-magnification objective lens 231 and a low-magnification objective lens 232. The high-magnification objective lens 231 is, for example, a 10x objective lens or a 20x objective lens. The low-magnification objective lens 232 is, for example, a 2x objective lens or a 4x objective lens. A combination of the high-magnification objective lens 231 and the low-magnification objective lens 232 is not limited to such combinations and is determined on the basis of a ratio of one magnification to the other magnification.

Here, the high-magnification objective lens 231, the fluorescent excitation light source 26, the transmission illumination system 27, a dichroic mirror 28, and the imaging unit 22 are included in a first optical system. The first optical system is an optical system for acquiring a main image at a high magnification. The main image is acquired using the high-magnification objective lens 231.

The low-magnification objective lens 232, the fluorescent excitation light source 26, the transmission illumination system 27, the dichroic mirror 28, and the imaging unit 22 are included in a second optical system. The second optical system is an optical system for acquiring a pre-image at a low magnification. The low magnification is a magnification lower than the magnification at which the main image is acquired. In other words, since a main image is acquired at a high magnification, the main image corresponds to a micro image or a high-magnification image. Since a pre-image is acquired at a low magnification, the pre-image corresponds to a macro image or a low-magnification image.

In the present embodiment, for example, the first optical system and the second optical system are provided as one detection optical system including a plurality of objective lenses in the microscope device 20. That is, the microscope device 20 includes the detection optical system (the first optical system and the second optical system) including a plurality of objective lenses such as the high-magnification objective lens 231 and the low-magnification objective lens 232.

The first optical system will be described below. The first optical system acquires a main image through fluorescence observation or transmissive bright-field observation. Fluorescence observation will be first described.

The fluorescent excitation light source 26 irradiates a cell C1 in a well W of the well plate WP with excitation light L1. The excitation light L1 emitted from the fluorescent excitation light source 26 is incident on the dichroic mirror 28. The excitation light L1 incident on the dichroic mirror 28 is reflected to the well plate WP by the dichroic mirror 28. The excitation light L1 reflected by the dichroic mirror 28 passes through the high-magnification objective lens 231 and is applied to the cell C1.

The cell irradiated with the excitation light L1 emits fluorescence L2. The fluorescence L2 passes through the high-magnification objective lens 231 and is incident on the dichroic mirror 28. The fluorescence L2 incident on the dichroic mirror 28 is transmitted to the imaging unit 22 by the dichroic mirror 28. The fluorescence L2 transmitted by the dichroic mirror 28 is focused on the imaging surface of the imaging unit 22 by a focusing lens which is not illustrated. The imaging unit 22 captures the focused image of the cell. The imaging unit 22 outputs the image captured by imaging the cell C1 to the analysis device 10.

Transmissive bright-field observation will be then described.

The transmission illumination system 27 irradiates a cell C1 with illumination light L3. The wavelength of the illumination light L3 is, for example, 720 nm but is not limited thereto. The illumination light L3 emitted to the cell C1 passes through the high-magnification objective lens 231 and is incident on the dichroic mirror 28. The illumination light L3 incident on the dichroic mirror 28 is transmitted to the imaging unit 22 by the dichroic mirror 28. The illumination light L3 transmitted by the dichroic mirror 28 is focused on the imaging surface of the imaging unit 22 by a focusing lens which is not illustrated. The imaging unit 22 captures a focused image of the cell C1. The imaging unit 22 outputs an image obtained by capturing the image of the cell to the analysis device 10. When transmissive bright-field observation is performed, the dichroic mirror 28 may be removed from an optical path.

The second optical system will be described below. The second optical system acquires a pre-image through transmissive bright-field observation or fluorescence observation. A method of acquiring a pre-image using the second optical system is the same as the method of acquiring a main image using the first optical system except that the low-magnification objective lens 232 instead of the high-magnification objective lens 231 is used as the objective lens, and thus description thereof will be omitted.

In the present embodiment, for example, it is assumed that the detection optical system provided in the microscope device 20 includes a plurality of objective lenses, but the present invention is not limited thereto. The first optical system and the second optical system may be provided as separate optical systems in the observation device 1. That is, the microscope device 20 may include a first optical system for acquiring a main image and a second optical system for acquiring a pre-image.

In the present embodiment, a cell image is acquired by expressing a protein tagged with fluorescence protein or staining a cell alive with a chemical reagent or the like. In another embodiment, a cell is fixed and stained, and then a cell image is acquired. Metabolism of a fixed cell stops. Accordingly, when an intracellular change with time is observed in a fixed cell after a stimulus has been given to the cell, it is necessary to prepare a plurality of containers in which cells have been seeded. For example, it may be intended to observe a change of a cell in a first time after a stimulus has been given to a cell and a change of the cell in a second time different from the first time. In this case, when the first time has elapsed after a stimulus has been given to a cell, the cell is fixed and stained, and then a cell image is acquired.

On the other hand, a container different from the container of the cell used for observation in the first time is prepared. When the second time has elapsed after a stimulus has been given to a cell, the cell is fixed and stained, and then a cell image is acquired. Accordingly, it is possible to estimate an intracellular change with time by observing a change of the cell in the first time and a change of the cell in the second time. The number of cells used to observe intracellular changes in the first time and the second time is not limited to one. As a result, images of a plurality of cells are acquired in the first time and the second time. For example, when the number of cells used to observe intracellular changes is 1000, 2000 cells are imaged in the first time and the second time. Accordingly, when it is intended to acquire details of an intracellular change in response to a stimulus, a plurality of cell images are necessary at each imaging timing from a stimulus, and a large number of cell images are acquired.

The microscope device 20 may image luminescence or fluorescence from a coloring matter taken into a biological material or luminescence or fluorescence generated through coupling of a matter having a chromophore to a biological material as the aforementioned cell image.

The method of acquiring a cell image is not limited to the optical microscope. For example, the method of acquiring a cell image may employ an electron microscope. That is, a type of a cell image may be appropriately selected.

A cell in the present embodiment is, for example, a primary cultured cell, an established cell strain, a cell of a tissue slice. A sample to be observed for the purpose of observation of a cell may be observed using a cell aggregate (a cell cluster, a spheroid, or an organoid), a tissue sample, an internal organ, or an individual (such as an animal), and an image including cells may be acquired. A cell state is not particularly limited and may be an alive state or a fixed state. Information of an alive state and information of a fixed state may be combined.

A cell may be processed using a chemical luminescent or fluorescent protein (for example, a chemical luminescent or fluorescent protein expressed from a transferred gene (such as a green fluorescent protein (GFP))) and may be observed. Alternatively, a cell may be observed using immunostaining or staining with a chemical reagent. A combination thereof may be used for observation. For example, a luminescent protein to be used may be selected according to a type of an intercellular structure (for example, an organelle (cell organelle) such as a Golgi apparatus).

A pre-process for analysis such as a means for observing cells or a method of staining cells may be appropriately selected according to the purpose. For example, dynamic information of a cell may be acquired using a technique optimal for obtaining dynamic behavior of a cell, and information on signal transmission in a cell may be acquired using a technique optimal for obtaining signal transmission in a cell. The pre-processes selected according to the purpose may be different.

The description of the functional configuration of the observation device 1 will be continued with reference back to FIG. 2.

The analysis device 10 is an analysis device that analyzes a cell image. For example, a response of a cell to a stimulus is analyzed from an image. In the present embodiment, the analysis device 10 is a computer device that determines an observation region in which a main image to be used for analysis is captured on the basis of the pre-image, images a cell in the determined observation region as a main image, and analyzes the main image.

The analysis device 10 includes an arithmetic operation unit 100, a storage unit 200, a result output unit 300, an operation detecting unit 400, and a control signal output unit 500.

The arithmetic operation unit 100 is realized by causing a processor to execute a program stored in the storage unit 200. Some or all of the functional units of the arithmetic operation unit 100 may be realized by hardware such as a large scale integration (LSI) circuit or an application-specific integrated circuit (ASIC). The arithmetic operation unit 100 includes a cell image acquiring unit 101, an image determining unit 102, an observation region selecting unit 103, an analysis unit 107, an auto-focusing unit 108, an abnormality detecting unit 110, an illumination condition determining unit 111, and an analysis type input unit 115 as functional units thereof.

The cell image acquiring unit 101 acquires a cell image from another device that captures an image. In this example, the cell image acquiring unit 101 acquires a cell image captured by the imaging unit 22 of the microscope device 20. The cell image acquiring unit 101 supplies the acquired cell image to an index calculation region changing unit 105, an index calculating unit 104, a region selecting unit 106, and an analysis unit 107.

Here, the cell image acquired from the microscope device 20 by the cell image acquiring unit 101 includes a main image, a pre-image, and a pre-ROI image and an overhead-view image which will be described later. An image includes pixels corresponding to the number of effective pixels of the imaging unit 22. A pixel resolution of a pre-image is lower than a pixel resolution of a main image. A region which is imaged as a pre-image is wider than a region which is imaged as a main image.

A pixel resolution is, for example, a size of a range in which an object is imaged per one pixel of an image (corresponding to one pixel of an imaging device). The pixel resolution can be represented by "(pixel resolution) = (size of imaging range of object)/(number of effective pixels of imaging device)" as an expression. The pixel resolution is also referred to as a resolution or a resolving power or a resolution.

An image acquired by the cell image acquiring unit 101 includes a plurality of images obtained by imaging a culture state of cells in a time series or a plurality of images obtained by culturing cells under various experimental conditions.

The image determining unit 102 performs various types of determination on images on the basis of image recognition. In the present embodiment, the image determining unit 102 determines a cell on the basis of a cell nucleus in an image, an outline of a cell in an image, or the like. The image determining unit 102 determines a type of the well plate WP or the like on the basis of a shape, a size, and the number of wells in an image. An image which is determined by the image determining unit 102 includes an overhead-view image, a pre-image, a pre-ROI image, and a main image.

Image recognition used for determination in the image determining unit 102 is, for example, image recognition based on artificial intelligence (AI). Pattern matching (template matching) or the like may be used for the image recognition.

The observation region selecting unit 103 determines an observation region for each well W. One observation region corresponds to each well W. The observation region is a so-called region of interest (ROI). The observation region selecting unit 103 includes an index calculating unit 104, an index calculation region changing unit 105, and a region selecting unit 106.

The index calculating unit 104 calculates a value of an index associated with a cell for each of subregions which are temporarily set in a pre-image by the index calculation region changing unit 105. Here, a subregion corresponds to a region to be used for analysis. In other words, a subregion included in a pre-image captured at a low magnification corresponds to a main image captured at a high magnification. An index of which a value is calculated by the index calculating unit 104 is an index based on a combination of a first index and a second index. That is, an index of which a value is calculated by the index calculating unit 104 includes a first index and a second index. Types of indices calculated by the index calculating unit 104 are stored as index information A1 in the storage unit 200 in advance. Any specific example of the index types will be described later. The index calculating unit 104 supplies the calculated value of the index to the region selecting unit 106.

The index calculation region changing unit 105 changes a subregion temporarily set in a pre-image in order to calculate an index value by the index calculating unit 104. The index calculation region changing unit 105 sequentially changes the subregion in the pre-image when the index calculating unit 104 calculates the index value. For example, the index calculation region changing unit 105 does not change the size of the subregion but changes a position of the subregion in the pre-image. Sequential changing of the subregion in the pre-image is also referred to as scanning of the pre-image. The index calculation region changing unit 105 supplies information of the changed subregion to the index calculating unit 104.

The region selecting unit 106 determines a region to be used for analysis out of a plurality of subregions on the basis of the index value calculated by the index calculating unit 104. That is, the region selecting unit 106 determines an observation region which is imaged at a high magnification as a main image for each well W on the basis of the index value calculated by the index calculating unit 104. The region selecting unit 106 selects the observation region, for example, on the basis of a combination of a value of the first index and a value of the second index. A specific example of the combination of the first index and the second index will be described later.

The analysis unit 107 acquires the main image from the cell image acquiring unit 101. The analysis unit 107 analyzes a cell in the main image acquired from the cell image acquiring unit 101. Analysis is, for example, cell-based assay such as measurement of protein expression increase/decrease in a cell, cell toxicity evaluation, evaluation of morphological changes in a cell, or measurement of an apoptotic cell proportion.

The analysis unit 107 supplies the analysis result to the result output unit 300.

The result output unit 300 acquires the analysis result from the analysis unit 107. The result output unit 300 displays the analysis result acquired from the analysis unit 107 on the display unit 30.

The operation detecting unit 400 detects an operation signal which is output from the operation unit 40. The operation detecting unit 400 supplies the detected operation signal to the arithmetic operation unit 100.

The analysis type input unit 115 receives an input of an analysis type. Here, a user inputs an analysis type by operating the operation unit 40. The operation unit 40 supplies the operation signal corresponding to the input analysis type to the analysis device 10. The analysis type input unit 115 acquires analysis type information on the basis of the operation signal supplied from the operation unit 40. The analysis type information is information indicating an analysis type.

The analysis type input unit 115 may acquire the analysis type information from an external device provided separate from the analysis device 10.

The index calculating unit 104 determines an index of which a value is calculated according to the analysis type information acquired by the analysis type input unit 115. The index calculating unit 104 determines the index on the basis of index information A1. The index information A1 is information in which an analysis type is associated with an index type. For example, the index information A1 is data of a two-dimensional table form including rows and columns in which index types are stored for each analysis type. The index information A1 is stored in the storage unit 200 in advance. The index information A1 may be prepared by allowing a user to operate the operation unit 40.

The storage unit 200 stores various types of information. The storage unit 200 stores, for example, the index information A1. The storage unit 200 is constituted by a storage device such as a magnetic hard disk device or a semiconductor storage device.

The types of the first index and the second index of which values are calculated by the index calculating unit 104 will be described below.

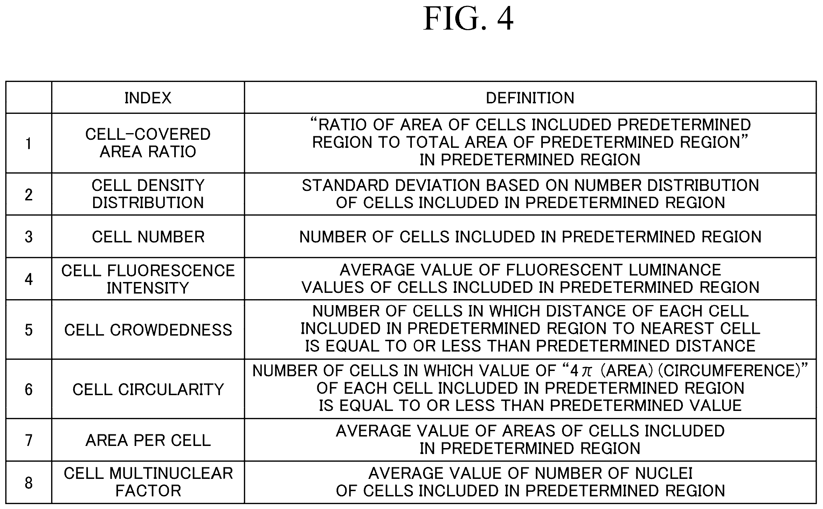

FIG. 4 is a diagram illustrating an example of index types according to the present embodiment. The first index and the second index are selected from the following indices (A) to (H):

(A) a cell-covered area ratio; (B) a cell density distribution; (C) a cell number; (D) a cell fluorescence intensity; (E) a cell crowdedness; (F) a cell circularity; (G) a area per cell; and (H) a cell multinuclear factor.

A case (a) and a case (b) can be considered as a method of determining an observation region of each well W using an index.

(a) case in which a subregion estimated to be substantially the same as the whole cell in a pre-image (a well W) is selected (representative value)

(b) case in which a subregion of which an index indicates one of a maximum value, a minimum value, an average value, and a predetermined % value (for example, a 75% value) (a value to be identified from a range from a maximum value to a minimum value) is selected from the whole cell in a pre-image (a well W).

In the case (a), it is necessary to calculate an absolute value of a difference between "an index value of each subregion" and "an index value of the whole pre-image." In the case (b), the "index value of each subregion" itself is used.

The cell-covered area ratio is a "ratio of an area of cells included in (occupying) a predetermined region to an area of the whole predetermined region" in the predetermined region. In order to the cell-covered area ratio, the area is calculated, for example, on the basis of the number of pixels in an image.

The cell density distribution is a standard deviation based on a distribution of the number of cells included in a predetermined region. That is, the cell density distribution is calculated on the basis of the number distribution of cells included in each minute region when the predetermined region is divided into minute regions.

The cell density distribution may be calculated on the basis of the k-nearest neighbors algorithm or the like instead of the aforementioned standard deviation.

The cell number is the number of cells included in a predetermined region.

The cell fluorescence intensity is an average value of fluorescent intensity of cells included in a predetermined region. That is, the cell fluorescence intensity is a value obtained by dividing a sum of the fluorescent intensity of cells included in the predetermined region by the number of cells included in the predetermined region.

The cell crowdedness is the number of cells in which a distance of each cell included in a predetermined region to a nearest cell is equal to or less than a predetermined distance. A distance between cells is a distance between the centers of gravity of cells. The center of gravity of a cell is a center when the outline of a cell is converted to a figure. The cell crowdedness may be an average value of the distances of the cells included in the predetermined region to the nearest cells.

The cell circularity is, for example, the number of cells in which a value calculated by Expression (1) for each cell included in a predetermined region is equal to or less than a predetermined value.

[Math. 1]

$$A = 4\pi \frac{(area)}{(circumferential\ length)^2} \quad (1)$$

That is, the value calculated by Expression (1) is a value obtained by multiplying a value obtained by dividing an area of a cell by a square of a circumferential length of the cell by $4\pi$. As the value calculated by Expression (1) becomes closer to 1, it means that the cell become more circular. In general, since a cell in cell division or a dead cell tends to be circular, the cell circularity is an index that can be used to simply detect the number of such cells.

The area per cell is an average value of areas of cells included in a predetermined region. That is, the area per cell is a value obtained by dividing the total area of the cells included in the predetermined region by the number of cells included in the predetermined region.

The cell multinuclear factor is an average of the numbers of nuclei of cells included in a predetermined region. That is, the cell multinuclear factor is a value obtained by dividing the total number of nuclei of the cells included in the predetermined region by the number of cells included in the predetermined region.

The aforementioned definitions of the indices are only examples, and the values of the indices may be evaluated by other definitions. The indices illustrated in FIG. 4 are only examples, and indices other than the indices illustrated in FIG. 4 may be used as the first index and the second index.

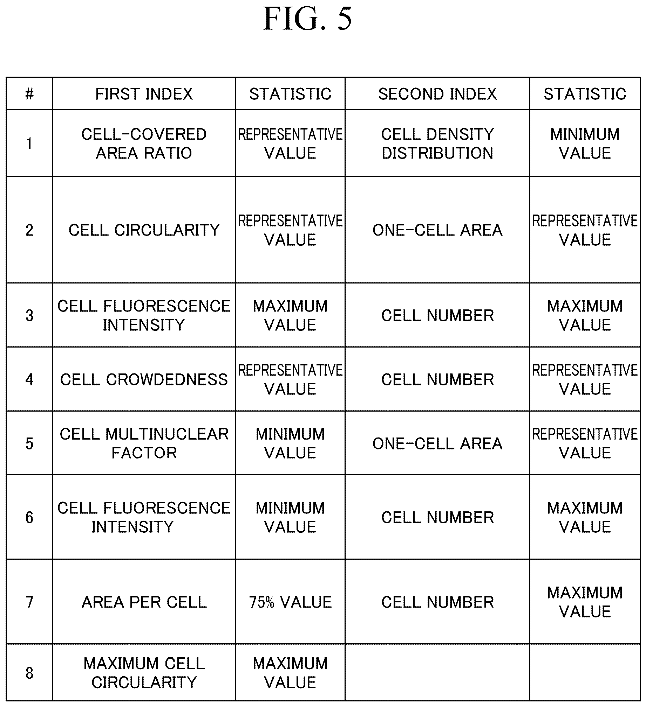

Combinations of the first index and the second index will be described below. FIG. 5 is a diagram illustrating an example of combinations of the first index and the second index according to the present embodiment. As will be described below, each of the first index and the second index is evaluated as a statistic of the scanned subregion in a pre-image and is used for the region selecting unit 106 to determine an observation region from the pre-image. As described above, an almost whole view of a well W is imaged in the pre-image. The statistic is, for example, a representative value, an average value, a maximum value, a minimum value, or a predetermined % value (for example, a 75% value).

A first combination is a combination of a representative value of the cell-covered area ratio and a minimum value of the cell density distribution. In order to identify the representative value of the cell-covered area ratio, an absolute value (referred to as an absolute value X11) of a difference between the "cell-covered area ratio of a subregion" and the "cell-covered area ratio in the whole pre-image" is calculated for each subregion. In order to identify the minimum value of the cell density distribution, a standard deviation (referred to as a standard deviation X12) based on the number distribution of cells included in a subregion is calculated. An index value based on the first combination is calculated, for example, as a product of the absolute value X11 and the standard deviation X12 for each subregion.

When the first combination is used, the region selecting unit 106 selects a subregion in which the index value based on the first combination is minimized as an observation region. That is, a subregion in which the cell-covered area ratio is estimated to be substantially the same as the whole cell in the pre-image and the cell density distribution is minimized is selected.

The index based on the first combination is appropriate for selecting a region in which the number of cells is representative and a region which is not affected by the cell density. That is, the index based on the first combination is effective for curbing variability in analysis depending on the method of selecting an observation region.

A second combination is a combination of a representative value of the cell circularity and a representative value of the area per cell. In order to identify the representative value of the cell circularity, an absolute value (referred to as an absolute value X21) of a difference between the "cell circularity of a subregion" and the "cell circularity in the whole pre-image" is calculated for each subregion. In order to identify the representative value of the area per cell, an absolute value (referred to as an absolute value X22) of a difference between the "area per cell of a subregion" and the "area per cell in the whole pre-image" is calculated for each subregion. An index value based on the second combination is calculated, for example, as a product of the absolute value X21 and the absolute value X22 for each subregion.

When the second combination is used, the region selecting unit 106 selects a subregion in which the index value based on the second combination is minimized as an observation region. That is, a subregion in which the cell circularity is estimated to be substantially the same as the whole cell in the pre-image and the area per cell is estimated to be substantially the same as the whole cell in the pre-image is selected.

The index based on the second combination is appropriate for analysis using parameters (such as a fluorescence intensity and the number) other than the shape because a cell with a presentative shape can be extracted. That is, the index based on the second combination is effective for curbing variability in analysis due to heterogeneity in cell morphology because the analysis can be performed except a cell with an abnormal shape. In other words, it is possible to reduce the load of an outlier removing operation after the analysis.

A third combination is a combination of a maximum value of the cell fluorescence intensity and a maximum value of the cell number. In order to identify the maximum value of the cell fluorescence intensity, a fluorescence intensity of a cell is calculated for each subregion. In order to identify the maximum value of the cell number, the cell number is calculated for each subregion. An index value based on the third combination is calculated, for example, as a product of the value of the cell fluorescence intensity and the value of the cell number for each subregion.

When the third combination is used, the region selecting unit 106 selects a subregion in which the index value based on the third combination is maximized as an observation region.

The index based on the third combination is appropriate for image analysis because a luminance value of a cell is high. The index based on the third combination is appropriate for acquiring as many cells as possible.

A fourth combination is a combination of a representative value of the cell crowdedness and a representative value of the cell number. In order to identify the representative value of the cell crowdedness, an absolute value (referred to as an absolute value X41) of a difference between the "cell crowdedness in a subregion" and the "degree of crowdedness in the whole pre-image" is calculated for each subregion. In order to identify the representative value of the cell number, an absolute value (referred to as an absolute value X42) of a difference between the "cell number in a subregion" and the "cell number in the whole pre-image" is calculated for each subregion. An index value based on the fourth combination is calculated, for example, as a product of the absolute value X41 and the absolute value X42 for each subregion.

When the fourth combination is used, the region selecting unit 106 selects a subregion in which the index value based on the fourth combination is minimized as an observation region. That is, a subregion in which the cell crowdedness is estimated to be substantially the same as the whole cell in the pre-image and a subregion in which the cell number is estimated to be substantially the same as the whole cell in the pre-image are selected.

Cells cultured in a well W include crowded cells and isolated cells, and a degree of crowdedness varies depending on the subregions. The index based on the fourth combination is appropriate for curbing variability in analysis due to heterogeneity in cell crowdedness.

A fifth combination is a combination of a minimum value of the cell multinuclear factor and a representative value of the area per cell. In order to identify the minimum value of the cell multinuclear factor, the cell multinuclear factor is calculated for each subregion. In order to identify the representative value of the area per cell, an absolute value (referred to as an absolute value X52) of a difference between the "area per cell in a subregion" and the "area per cell in the whole pre-image" is calculated for each subregion. An index value based on the fifth combination is calculated, for example, as a product of the value of the cell multinuclear factor and the absolute value X52 for each subregion.

When the fifth combination is used, the region selecting unit 106 selects a subregion in which the index value based on the fifth combination is minimized as an observation region.

The index based on the fifth combination is appropriate for analysis except multinuclear cells.

A sixth combination is a combination of a minimum value of the cell fluorescence intensity and a maximum value of the cell number. In order to identify the minimum value of the cell fluorescence intensity, the cell fluorescence intensity is calculated for each subregion. In order to identify the maximum value of the cell number, the cell number is calculated for each subregion. An index value based on the sixth combination is calculated, for example, as a value obtained by dividing the value of the cell number by the value of the cell fluorescence intensity for each subregion.

When the sixth combination is used, the region selecting unit 106 selects a subregion in which the index value based on the sixth combination is maximized as an observation region.

The index based on the sixth combination is appropriate for increasing the number of cells as many as possible in a region in which fluorescence is not generated from a fluorescent dye staining a dead cell. For example, the index based on the sixth combination is appropriate for excluding dead cells when a reagent is administrated and analyzing only living cells by staining the dead cells and excluding the dead cells from analysis.

A seventh combination is a combination of a 75% value of the area per cell and a maximum value of the cell number. In order to identify the 75% value of the area per cell, the one-cell area is calculated for each subregion. The 75% value is calculated on the basis of the value of the area per cell of each subregion using a known 75% value calculating method. In order to identify the maximum value of the cell number, the cell number is calculated for each subregion. In order to calculate the index value based on the seventh combination, first, for example, what % value the "area per cell" for each subregion corresponds to is calculated, and a value (referred to as a relative value X71) obtained by dividing a difference between the calculated value (X%) of the subregion and 75% by 100% is calculated for each subregion. Then, a value (referred to as a relative value X72) obtained by dividing a difference between the cell number for each subregion and the "maximum value of the cell number" by the "maximum value of the cell number" is calculated. The index value based on the seventh combination is calculated as a product of the relative value X71 and the relative value X72.

When the seventh combination is used, the region selecting unit 106 selects a subregion in which the index value based on the seventh combination is minimized as an observation region.

The index based on the seventh combination is appropriate for acquiring cells with a large area, excluding an outlier as much as possible, and acquiring the cells as many as possible.

An eighth combination is a maximum value of the cell circularity. In the eighth combination, the first index and the second index are not combined, and one index is used. In order to identify the maximum value of the cell circularity, the cell circularity is calculated for each subregion. The index value based on the eighth combination is the cell circularity itself.

When the eighth combination is used, the region selecting unit 106 selects a subregion in which the index value based on the eighth combination is maximized as an observation region.

The index based on the eighth combination is appropriate for extracting a cell in a mitotic phase. A cell with a large circularity is likely to be a dead cell.

In a combination of indices in which a product of a statistic of the first index and a statistic of the second index is evaluated, a product of weighted sums may be evaluated instead of the product. For example, in the first combination, the index value based on the first combination is calculated as the product of the absolute value X11 and the standard deviation X12 as described above. The index value based on the first combination may be calculated as a sum of the weighted absolute value X11 (aX11) and the weighted standard deviation X12 (bX12). The same is true of the indices based on the second combination, the third combination, the fourth combination, the fifth combination, and the seventh combination.

The index value based on the sixth combination may be calculated as a difference between the weighted value of the cell number and the weighted value of the cell fluorescence intensity.

In the present embodiment, it is assumed that the index is a combination of the first index and the second index, but the present invention is not limited thereto. One index may be used as the index. For example, the index may be one of 8 indices illustrated in FIG. 4.

The index may be a combination of three or more indices.

[Imaging process]

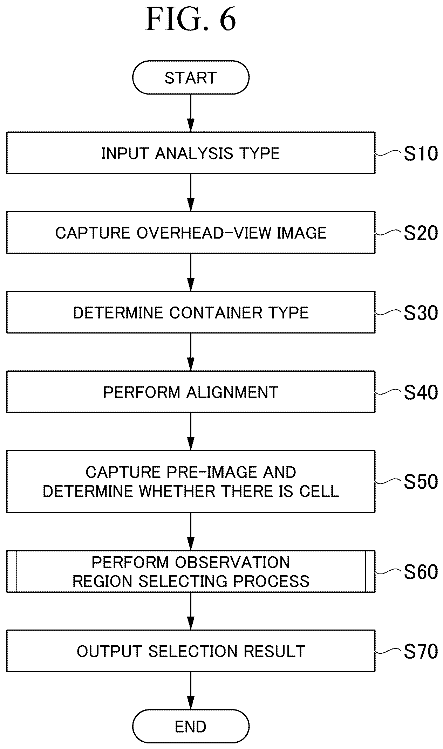

A process flow in which the observation device 1 captures a cell image and analyzes the captured cell image will be described below with reference to FIGS. 6 to 10. The process flow that the observation device 1 performes includes a first preparation process, a second preparation process, and a main image capturing and analyzing process in this order.

FIG. 6 is a diagram illustrating an example of a flow of the first preparation process according to the present embodiment. The first preparation process is a process of determining an observation region using a pre-image.

Step S10: The analysis type input unit 115 receives an analysis type as an input. A user inputs the analysis type by operating the operation unit 40. The analysis type input unit 115 acquires analysis type information on the basis of the operation signal supplied from the operation unit 40.

Thereafter, the analysis device 10 performs the process of Step S20.

Step S20: The microscope device 20 captures an overhead-view image of the whole or a most part of the well plate WP. The microscope device 20 captures an overhead-view image through transmissive bright-field observation using the second optical system. The cell image acquiring unit 101 acquires the overhead-view image. The cell image acquiring unit 101 supplies the overhead-view image to the image determining unit 102.

Thereafter, the analysis device 10 performs the process of Step S30.

Step S30: The image determining unit 102 determines a type (container type) of the well plate WP. For example, the image determining unit 102 determines the type (container type) of the well plate WP on the basis of the shape, the size, the number, and the like of the wells W imaged in the overhead-view image through image recognition. Here, the image determining unit 102 uses the magnification at which the overhead-view image is captured to determine the size of the wells W.

Thereafter, the analysis device 10 performs the process of Step S40.

Step S40: The image determining unit 102 performs alignment of the well plate WP using the overhead-view image and corrects (acquires) information of the center position of each well in the well plate WP.

Thereafter, the analysis device 10 performs the process of Step S50.

Step S50: The microscope device 20 captures a pre-image for each well W through transmissive bright-field observation using the second optical system. The image determining unit 102 determines whether a cell is included in the pre-image. The image determining unit 102 determines a part corresponding to the cell from the pre-image through image recognition.

Thereafter, the analysis device 10 performs the process of Step S60.

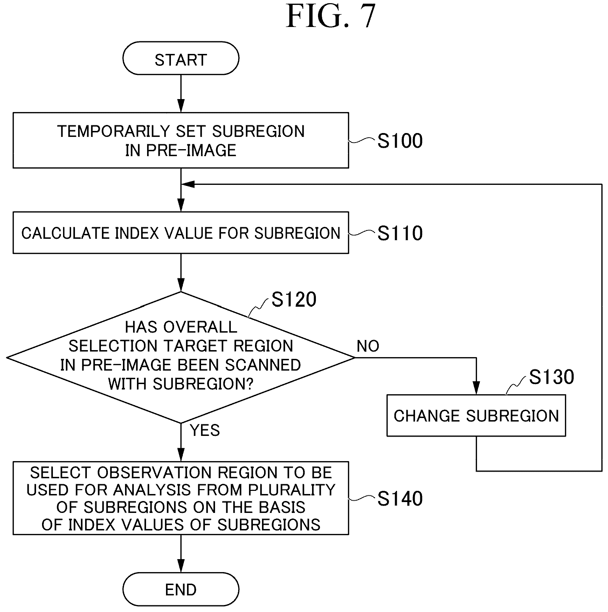

Step S60: The observation region selecting unit 103 performs an observation region selecting process of determining an observation region for each well W of a plurality of wells W including a cell. Details of the observation region selecting process will be described later with reference to FIG. 7.

The observation region selecting process will be described below with reference to FIG. 7. FIG. 7 is a diagram illustrating an example of a flow of the observation region selecting process according to the present embodiment. As described above, the observation region selecting process illustrated in FIG. 7 is performed as the process of Step S60 by the observation region selecting unit 103.

Step S100: The index calculation region changing unit 105 temporarily sets a subregion in the pre-image for each well W.

Thereafter, the observation region selecting unit 103 performs the process of Step S110.

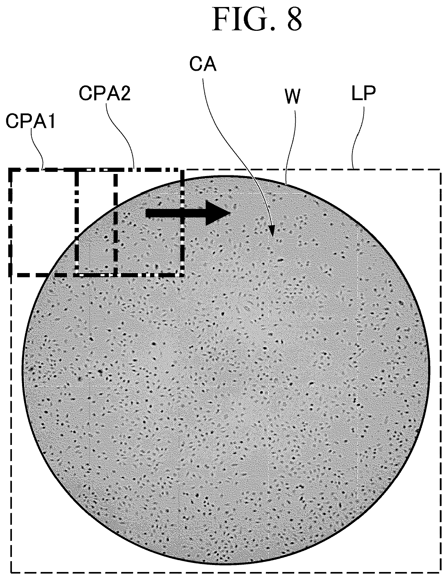

A pre-image will be described below with reference to FIG. 8. FIG. 8 is a diagram illustrating an example of a pre-image according to the present embodiment. In FIG. 8, a pre-image LP is illustrated aa an example of the pre-image acquired by the cell image acquiring unit 101. In this example, the pre-image LP is an image in which an almost overall view of the well W is imaged as described above.

A seeding region CA which is a region to which a cell is seeded is included in the well W. In the seeding region CA, cells cannot be uniformly seeded due to various conditions. Cells in the seeding region CA may not be uniformly distributed, for example, because the cells are cells after culture, a culturing direction of the cells is not constant in a culturing period, and the cells may be cultured, for example, in a specific direction. Accordingly, the seeding region CA includes a region including a smaller number of cells or a region which is not appropriate for analysis due to overlap of the cells.

A subregion CPA1 is an example of a subregion which is set by the index calculation region changing unit 105.

Description of the observation region selecting process will be continued with reference back to FIG. 7.

Step S110: The index calculating unit 104 calculates a value of an index in the subregion which is temporarily set by the index calculation region changing unit 105. Here, the index calculating unit 104 reads the index information A1 from the storage unit 200 and determines an index of which the value is calculated on the basis of the read index information A1 according to the analysis type information acquired by the analysis type input unit 115. The index information A1 includes, for example, the first to eighth combinations.

In the present embodiment, for example, it is assumed that the index of which the value is calculated is automatically determined, but the present invention is not limited thereto. For example, in the process of Step S10 (inputting the analysis type) in FIG. 6 or after the process of Step S50 in FIG. 6 has ended and before the process of Step S60 has started, an input unit for inputting an index of which the value is calculated may be provided such that a user can input a type of the index of which the value is calculated by operating the operation unit 40.

For example, when the index determined according to the analysis type information is the first combination, the index calculating unit 104 calculates the product of the absolute value X11 and the standard deviation X12. As described above, the absolute value X11 is an absolute value of a difference between the cell-covered area ratio for each subregion and the cell-covered area ratio in the whole pre-image. The standard deviation X12 is a standard deviation of the number distribution of cells included in a minute region to which the subregion is further divided. The index calculating unit 104 calculates the values of the absolute value X11 and the standard deviation X12 on the basis of a result of image recognition of the subregion by the image determining unit 102. The values of the absolute value X11 and the standard deviation X12 may be calculated by the image determining unit 102 and supplied to the index calculating unit 104.

Thereafter, the observation region selecting unit 103 performs the process of Step S120.

Step S120: The index calculation region changing unit 105 determines whether an overall selection target region in the pre-image has been scanned with the subregion. The selection target region is a region which is scanned with the subregion in the pre-image. The selection target region may be the whole pre-image or a remaining part obtained by excluding a peripheral part from the whole pre-image.

When the index calculation region changing unit 105 determines that the overall selection target region in the pre-image has been scanned with the subregion (Step S120: YES), the observation region selecting unit 103 performs the process of Step S140. When the index calculation region changing unit 105 determines that the overall selection target region in the pre-image has not been scanned with the subregion (Step S120: NO), the observation region selecting unit 103 performs the process of Step S130.

Step S130: The index calculation region changing unit 105 changes the subregion. The index calculation region changing unit 105 changes the subregion, for example, by translating (shifting) the subregion by a predetermined position in the X direction in the pre-image. When the subregion deviates from the selection target region as the result of translation, the index calculation region changing unit 105 translates the subregion by a predetermined position in the Y direction in the pre-image. Thereafter, the index calculation region changing unit 105 translates the subregion by a predetermined position in the X direction in the pre-image again. That is, the index calculation region changing unit 105 performs canning in the X direction while changing the position in the Y direction.

The index calculation region changing unit 105 may perform scanning in the Y direction while changing the position in the X direction. The selection order of the subregions is not particularly limited.

When the index calculation region changing unit 105 changes the subregion, a post-change subregion and a previously selected subregion may have an overlap part. The index calculation region changing unit 105 may change the subregion such that the post-change subregion and the previously selected subregion do not overlap.

In the example illustrated in FIG. 8, the index calculation region changing unit 105 changes the subregion from the subregion CPA1 to the subregion CPA2.

The description of the observation region selecting process will be continued with reference back to FIG. 7.

Thereafter, the observation region selecting unit 103 performs the process of Step S110 again.

Step S140: The region selecting unit 106 selects (determines) an observation region to be used for analysis from a plurality of subregions on the basis of the index values of the observation regions calculated by the index calculating unit 104. In the present embodiment, the region selecting unit 106 selects one observation region to be used for analysis from the plurality of subregions on the basis of the index values calculated by the index calculating unit 104. In this case, for example, when the first combination is selected from the index information A1, the region selecting unit 106 selects a subregion in which the index value based on the first combination is minimized as the observation region.

The region selecting unit 106 selects a subregion in which an evaluation value based on the index value calculated by the index calculating unit 104 is the highest. For example, when the index type is the first combination, the region selecting unit 106 selects a subregion in which the index value based on the first combination is minimized as the observation region.

The region selecting unit 106 outputs the observation region for each well W to the result output unit 300.

In this way, the observation region selecting unit 103 ends the observation region selecting process.

Description of the first preparation process will be continued with reference back to FIG. 6.

Thereafter, the result output unit 300 performs the process of Step S70.

Step S70: The result output unit 300 displays the result (the selection result of an observation region) supplied from the region selecting unit 106 on the display unit 30.

The display unit 30 arranges and displays a plurality of pre-images, for example, to correspond to the addresses of the wells W in the well plate WP and superimposes and displays the observation regions of the wells W on the pre-images. A plurality of wells W may be displayed as a top view of the well plate WP, and the observation regions of the wells W may be displayed to overlap the wells W. Since the observation region is set for only a well W in which there is a cell, the observation region is not displayed in the pre-image in which there is no cell and the well W in which there is no cell.

In this way, the observation device 1 ends the first preparation process.

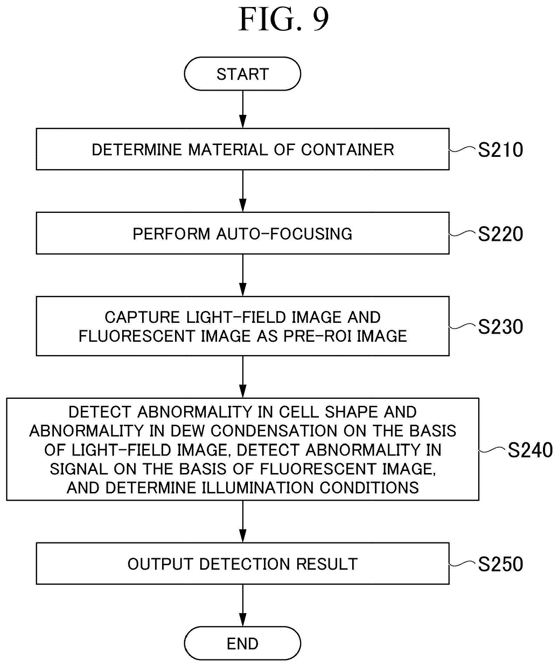

The second preparation process will be described below with reference to FIG. 9. FIG. 9 is a diagram illustrating an example of a flow of the second preparation process according to the present embodiment. The second preparation process is performed when the user checks the result displayed on the display unit 30 in the process of Step S70 (FIG. 6), determines whether to perform the second preparation process, and presses a "button to next" which is not illustrated by operating the operation unit 40 after the first preparation process has ended.

Step S210: Laser light from a laser light source which is not illustrated in FIG. 3 is emitted to the bottom surface of the well plate, an intensity of reflected light is detected by a detector which is not illustrated, and the material of the well plate WP is determined on the basis of the intensity.

Thereafter, the analysis device 10 performs the process of Step S220.

Step S220: The auto-focusing unit 108 performs an auto-focusing process. The auto-focusing unit 108 determines an observation position in the Z-axis direction for each observation region of each well W through the auto-focusing process. The auto-focusing unit 108 selects an auto-focusing algorithm according to the material of the container.

For example, when the material of the container is glass, the auto-focusing unit 108 selects an auto-focusing (image contrast AF) algorithm based on an image contrast. Instead, a method of offsetting a predetermined amount in the optical axis direction may be selected by performing auto-focusing on the bottom surface (the interface) of the well plate WP. For example, when the material of the container is plastic, the auto-focusing unit 108 selects an auto-focusing algorithm based on AI. The auto-focusing algorithm to be applied is selected according to the material of the well plate WP.

Here, the control signal output unit 500 sets a focal position of the objective lens 23. The control signal output unit 500 sets the focal position of the objective lens 23 at the observation position in the Z-axis direction determined by the auto-focusing unit 108. The control signal output unit 500 supplies a control signal for moving the objective lens 23 such that the focal position of the objective lens 23 matches the observation position in the Z-axis direction to the objective lens drive unit 24. The objective lens drive unit 24 drives the objective lens 23 according to the control signal.

Thereafter, the observation device 1 performs the process of Step S230.

Step S230: The microscope device 20 captures a bright-field image and a fluorescent image as a pre-ROI image at the observation position in the Z-axis direction for each observation region in each observation region of each well W. The microscope device 20 captures a bright-field image through transmissive bright-field observation using the first optical system (high magnification) and captures a fluorescent image through fluorescence observation using the first optical system (high magnification). When a fluorescent image is not captured as a main image which will be described later, the fluorescent image does not need to be captured as the pre-ROI image.

The cell image acquiring unit 101 acquires the bright-field image and the fluorescent image.

Thereafter, the analysis device 10 performs the process of Step S240.

Step S240: The abnormality detecting unit 110 determines whether an abnormality of a cell shape or an abnormality of dew condensation is included in the bright-field image. The abnormality detecting unit 110 determines whether an abnormality of a signal is included in the fluorescent image.

The abnormality detecting unit 110 outputs information indicating whether an abnormality of a cell shape or dew condensation is included in the bright-field image and whether an abnormality of a signal is included in the fluorescent image to the result output unit 300. The illumination condition determining unit 111 determines illumination conditions (such as an illumination light power) on the basis of the fluorescent image and outputs the determination result to the result output unit 300.

Thereafter, the analysis device 10 performs the process of Step S250.

Step S250: The result output unit 300 displays the results (detection results) supplied from the abnormality detecting unit 110 and the illumination condition determining unit 111 on the display unit 30.

In this way, the observation device 1 ends the second preparation process.

When the region selecting unit 106 selects a plurality of subregions as candidates for the observation region of each well W on the basis of the index value calculated by the index calculating unit 104, the processes of Step S220 to S240 are performed on the plurality of subregions, and results thereof are displayed on the display unit 30. In this case, a user determines the observation region of each well W by selecting one of the candidates for the observation region of each well W.

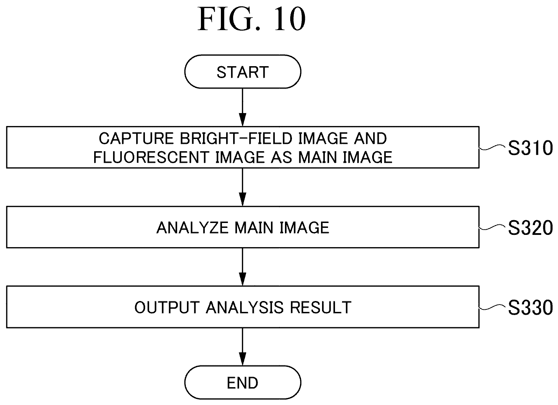

The main image capturing and analyzing process will be described below with reference to FIG. 10. FIG. 10 is a diagram illustrating an example of a flow of the main image capturing and analyzing process according to the present embodiment. The main image acquisition and analyzing process is performed when the user checks the result displayed on the display unit 30 in the process of Step S250, determines whether to perform the main image acqusition and analyzing process, and presses a "button to next" which is not illustrated by operating the operation unit 40 after the second preparation process has ended.

Step S310: The microscope device 20 captures a bright-field image and a fluorescent image as a main image at the observation position in the Z-axis direction for each observation region in each observation region of each well W. The microscope device 20 captures a bright-field image through transmissive bright-field observation using the first optical system (high magnification) and acquires a fluorescent image through fluorescence observation using the first optical system (high magnification) under the illumination conditions determined by the illumination condition determining unit 111.

The cell image acquiring unit 101 acquires the bright-field image and the fluorescent image. The cell image acquiring unit 101 supplies the main image to the analysis unit 107.

Thereafter, the analysis device 10 performs the process of Step S320.

Step S320: The analysis unit 107 analyzes the main mage acquired from the cell image acquiring unit 101. The analysis unit 107 supplies the analysis result to the result output unit 300.

Thereafter, the analysis device 10 performs the process of Step S330.

Step S330: The result output unit 300 displays the analysis result supplied form the analysis unit 107 on the display unit 30.

In this way, the observation device 1 ends the main image acquisitionand analyzing process.

In the present embodiment, the index type is determined according to the analysis type the observation region selecting process for each well W, but only a group of indices (a combination of two types of indices, for example, the first combination) stored in advance in the storage unit 200 may be used.

The observation region of each well W is automatically determined by the observation region selecting unit 103, but the present invention is not limited thereto. For example, an observation region determination method input unit for allowing a user to select one of a process (c) and a process (d) by operating the operation unit 40 may be provided in the process of Step S10 (inputting an analysis type) in FIG. 6.

   (c) Automatic determination by the observation region selecting unit 103
   (d) targeting determination of a predetermined region (for example, the central region of the well W)

When the process (d) is input to the observation region determination method input unit, the process flow illustrated in FIG. 7 is not performed, and the observation region selecting unit 103 determines a region predetermined for each of a plurality of wells W including cells and then performs the process of Step S70 illustrated in FIG. 6.

As described above, the observation device 1 according to the present embodiment is an observation device that analyzes a cell image and includes an image acquiring unit (the microscope device 20 in the present embodiment), the index calculating unit 104, and the region selecting unit 106.

The image acquiring unit (the microscope device 20 in the present embodiment) acquires a second image (a pre-image in the present embodiment) in a region wider than a region used for the analysis at a pixel resolution lower than a pixel resolution of a first image (a main image in the present embodiment) to be used for analysis.

The index calculating unit 104 calculates a value of an index for a cell in each of the subregions included in the second image (the pre-image in the present embodiment) and corresponding to the region to be used for analysis.

The region selecting unit 106 selects a region (an observation region in the present embodiment) to be used for analysis from the plurality of subregions on the basis of the value of the index calculated by the index calculating unit 104.

With this configuration, in the observation device 1 according to the present embodiment, since the region to be used for analysis (the observation region in the present embodiment) can be selected out of the subregions on the basis of the value of the index calculated for each subregion included in the second image (the pre-image in the present embodiment), it is possible to curb arbitrary selection of the region to be used for analysis (the observation region in the present embodiment). Accordingly, in the observation device 1 according to the present embodiment, it is possible to curb variability in analysis result due to selection of the region to be used for analysis.

In high content analysis (HCA), imaging is performed for each well, and quantitative analysis is performed. The analysis result may vary according to an algorithm of selecting an observation region in a well. For example, since there are various types of behavior of cells in one well, the algorithm of selecting an observation region in a well can be a cause of variability in analysis result. When a region including a small number of cells is selected as an observation region, there is a likelihood that AF will not be performed or statistical analysis cannot be performed.

In the related art, one well is observed, and an observation region to be used for imaging and analysis is determined. When one well is observed and an observation region is determined, it requires much time. In the related art, the observation region is determined as the central region of a well, a plurality of wells with the same conditions are prepared, and wells statistically having an outlier are excluded from analysis. In this case, the cost of reagents increases, and the number of experiment times increases.

On the other hand, in the observation device 1 according to the present embodiment, a region most appropriate for observation can be selected from an observation target. The region most appropriate for observation is, for example, a representative position in consideration of various types of behavior of cells in one well.

In the observation device 1 according to the present embodiment, the index includes a first index and a second index (the index is a combination of the first index and the second index in the present embodiment).

With this configuration, in the observation device 1 according to the present embodiment, since the region to be used for analysis can be selected on the basis of the index including the first index and the second index, it is possible to select a more appropriate region in comparison with a case in which the region to be used for analysis is selected on the basis of a value of one index.

In the observation device 1 according to the present embodiment, the first index and the second index are selected from the following indices (A) to (H):
(A) a cell-covered area ratio; (B) a cell density distribution; (C) a cell number; (D) a cell fluorescence intensity; (E) a cell crowdedness; (F) a cell circularity; (G) a area per cell; and (H) a cell multinuclear factor.

With this configuration, in the observation device 1 according to the present embodiment, since the region to be used for analysis can be selected on the basis of the first index and the second index selected from the indices (A) to (H), it is possible to select a region corresponding to the purpose of analysis.

The observation device 1 according to the present embodiment includes another input unit (the analysis type input unit 115 in the present embodiment).

The input unit (the analysis type input unit 115 in the present embodiment) receives an analysis type as an input.

The analysis type input unit 115 determines the index of which the value is to be calculated according to the analysis type.

In the observation device 1 according to the present embodiment, the image acquiring unit (the microscope device 20 in the present embodiment) includes a detection optical system (the first optical system and the second optical system in the present embodiment) including a plurality of objective lens (the high-magnification objective lens 231 and the low-magnification objective lens 232 in the present embodiment).

The first image (the main image in the present embodiment) is acquired using a first objective lens (the high-magnification objective lens 231 in the present embodiment) out of the plurality of objective lenses.

The second image (the pre-image in the preset embodiment) is acquired using a second objective lens (the low-magnification objective lens 232 in the present embodiment) with a magnification lower than that of the first objective lens (the high-magnification objective lens 231 in the present embodiment) out of the plurality of objective lenses.

With this configuration, in the observation device 1 according to the present embodiment, the configuration is more simple than that in the case in which an optical system for acquiring the first image (the main image in the present embodiment) and an optical system for acquiring the second image (the pre-image in the present embodiment) are provide as separate optical systems in the image acquiring unit (the microscope device 20 in the present embodiment).

In the observation device 1 according to the present embodiment, the image acquiring unit (the microscope device 20 in the present embodiment) may include a first optical system for acquiring the first image (the main image in the present embodiment) and a second optical system for acquiring the second image (the pre-image in the present embodiment).

With this configuration, in the observation device 1 according to the present embodiment, desired imaging conditions (magnifications) can be more easily realized in comparison with the case in which an optical system for acquiring the first image (the main image in the present embodiment) and an optical system for acquiring the second image (the pre-image in the present embodiment) is provided as a unified optical system.

(Second embodiment)

Hereinafter, a second embodiment of the present invention will be described in detail with reference to the drawings.

In the first embodiment, an example in which an index is determined according to the analysis type has been described. In the present embodiment, it is assumed that the index is determined from the cell type.

The observation device 1 according to the present embodiment is referred to as an observation device 1a, and the analysis device is referred to as an analysis device 10a.

The same constituents as in the first embodiment will be referred to by the same reference signs, and description of the same constituents and operations may be omitted.

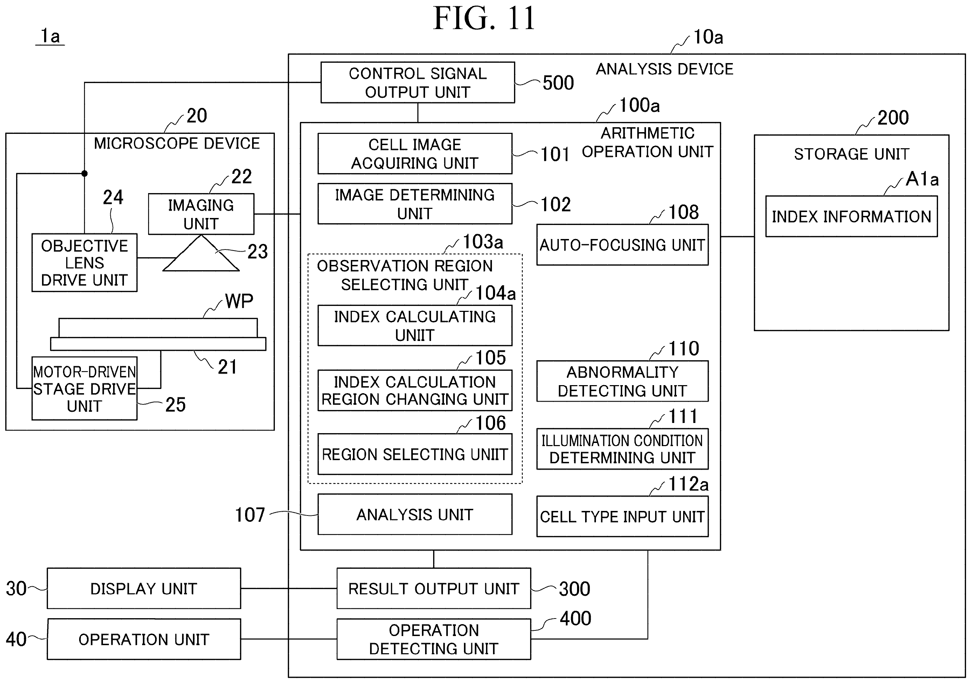

FIG. 11 is a block diagram illustrating an example of a functional configuration of the observation device 1a according to the present embodiment. The observation device 1a includes an analysis device 10a, a microscope device 20, a display unit 30, and an operation unit 40. The observation device 1a (FIG. 11) according to the present embodiment is different from the observation device 1 (FIG. 2) according to the first embodiment in the analysis device 10a. On the other hand, the functions of the other constituents (the microscope device 20, the display unit 30, and the operation unit 40) are the same as those in the first embodiment.

The analysis device 10a includes an arithmetic operation unit 100a, a storage unit 200, a result output unit 300, an operation detecting unit 400, and a control signal output unit 500. The arithmetic operation unit 100a (FIG. 11) according to the present embodiment is different from the arithmetic operation unit 100 (FIG. 2) according to the first embodiment in an observation region selecting unit 103a and a cell type input unit 112a. On the other hand, the functions of the other constituents (the cell image acquiring unit 101, the image determining unit 102, the analysis unit 107, the auto-focusing unit 108, the abnormality detecting unit 110, and the illumination condition determining unit 111) are the same as those in the first embodiment.

The cell type input unit 112a receives a cell type as an input. Here, a user inputs a cell type by operating the operation unit 40. The operation unit 40 supplies the operation signal corresponding to the input cell type to the analysis device 10. The cell type input unit 112a acquires cell type information on the basis of the operation signal supplied from the operation unit 40. The cell type information is information indicating a cell type.

The cell type input unit 112a may acquire the cell type information from an external device provided separate from the analysis device 10a.

The observation region selecting unit 103a includes an index calculating unit 104a, an index calculation region changing unit 105, and a region selecting unit 106. The functions of the index calculation region changing unit 105 and the region selecting unit 106 are the same as those in the first embodiment.

The index calculating unit 104a determines an index of which a value is calculated according to the cell type information acquired by the cell type input unit 112a. The index calculating unit 104a determines the index on the basis of index information A1a. The index information A1a is information in which a cell type and an index type are correlated. For example, the index information A1a is data of a two-dimensional table form including rows and columns in which index types are stored for each cell type. The index information A1a is stored in the storage unit 200 in advance. The index information A1a may be prepared by allowing a user to operate the operation unit 40.

[Imaging process]

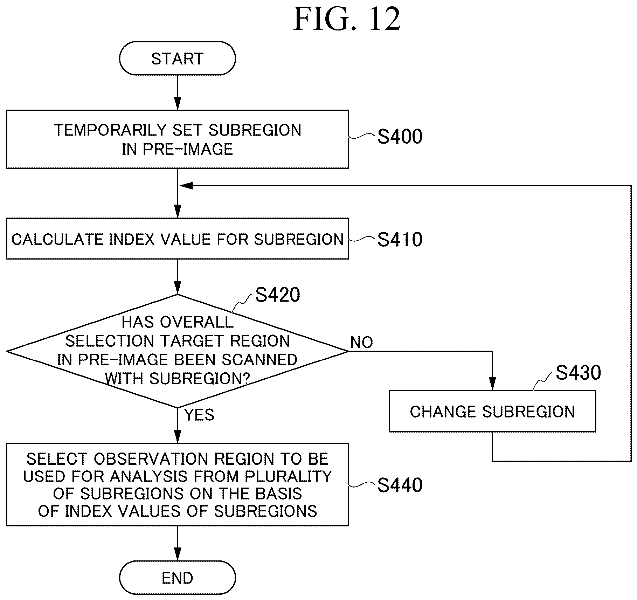

An imaging process that is performed by the observation device 1a will be described below with reference to FIG. 12. The imaging process performed by the observation device 1a is different from the imaging process performed by the observation device 1 according to the first embodiment in an observation region selecting process included in the first preparation process.

In the imaging process performed by the observation device 1a, the cell type input unit 112a inputs the cell type instead of the process of causing the analysis type input unit 115 to input the analysis type in the process of Step S10 in FIG. 6. A user inputs a cell type by operating the operation unit 40. The cell type input unit 112a acquires cell type information on the basis of the operation signal supplied from the operation unit 40.

The analysis type input unit 115 performs the process of inputting an analysis type, and the cell type input unit 112a may additionally perform the process of inputting a cell type.

FIG. 12 is a diagram illustrating an example of a flow of the observation region selecting process according to the present embodiment. The observation region selecting process illustrated in FIG. 12 is performed as the process of Step S60 in FIG. 6 by the observation region selecting unit 103a.

The processes of Steps S400, S420, S430, and S440 are the same as the processes of Steps S100, S120, S130, and S140 in FIG. 7, and thus a description thereof will be omitted.

Step S410: The index calculating unit 104a calculates a value of an index for a subregion which is temporarily set by the index calculation region changing unit 105. The index calculating unit 104a determines an index of which the value is calculated according to the cell type information acquired by the cell type input unit 112a. Here, the index calculating unit 104a reads the index information A1a from the storage unit 200 and determines an index correlated with the cell type information on the basis of the read index information A1a.

Thereafter, the analysis device 10a performs the process of Step S420.

As described above, the observation device 1a according to the present embodiment includes another input unit (the cell type input unit 112a in the present embodiment).

The other input unit (the cell type input unit 112a in the present embodiment) receives a cell type as an input.

The cell type input unit 112a determines the index of which the value is to be calculated according to the cell type.

With this configuration, in the observation device 1a according to the present embodiment, since a region to be used for analysis can be selected from a plurality of subregions on the basis of the index corresponding to the cell type, it is possible to select a region appropriate for analysis in comparison with a case in which the region is selected on the basis of a predetermined index.

(Third embodiment)

Hereinafter, a second embodiment of the present invention will be described in detail with reference to the drawings.

In the present embodiment, it is assumed that a predetermined value is added to or subtracted from a value of an index for a subregion in which foreign matter is imaged when the subregion is included in a pre-image.

The observation device according to the present embodiment is referred to as an observation device 1b, and the analysis device is referred to as an analysis device 10b.

The same constituents as in the first embodiment will be referred to by the same reference signs, and a description of the same constituents and operations may be omitted.

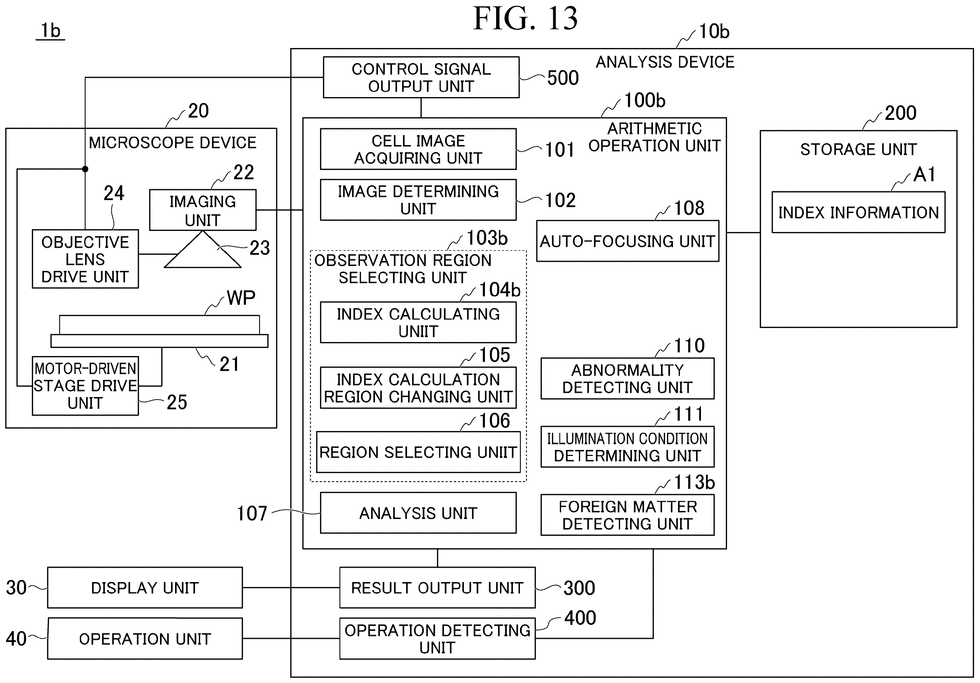

FIG. 13 is a block diagram illustrating an example of a functional configuration of the observation device 1b according to the present embodiment. The observation device 1b includes an analysis device 10b, a microscope device 20, a display unit 30, and an operation unit 40. The observation device 1b (FIG. 13) according to the present embodiment is different from the observation device 1 (FIG. 2) according to the first embodiment in the analysis device 10b. On the other hand, the functions of the other constituents (the microscope device 20, the display unit 30, and the operation unit 40) are the same as those in the first embodiment.

The analysis device 10b includes an arithmetic operation unit 100b, a storage unit 200, a result output unit 300, an operation detecting unit 400, and a control signal output unit 500. The arithmetic operation unit 100b (FIG. 13) according to the present embodiment is different from the arithmetic operation unit 100 (FIG. 2) according to the first embodiment in an observation region selecting unit 103b and foreign matter detecting unit 113b. On the other hand, the functions of the other constituents (the cell image acquiring unit 101, the image determining unit 102, the analysis unit 107, the autofocusing unit 108, the abnormality detecting unit 110, and the illumination condition determining unit 111) are the same as those in the first embodiment.

The foreign matter detecting unit 113b detects foreign matter when the foreign matter is imaged in the pre-image. The foreign matter is, for example, a scar attached to the surface of a well W or dust or waste mixed into the well W. A subregion in which foreign matter in the pre-image is imaged is a region to be excluded from an observation region. In general, luminance of the foreign matter in the captured image is higher than luminance of a cell. Accordingly, for example, the foreign matter may be erroneously recognized as a cell, and analysis may be performed thereon.

The observation region selecting unit 103b includes an index calculating unit 104b, an index calculation region changing unit 105, and a region selecting unit 106. The functions of the index calculation region changing unit 105 and the region selecting unit 106 are the same as those in the first embodiment.

When the region selecting unit 106 selects a subregion with a small index value as a region to be used for analysis and there is a subregion in which foreign matter is imaged, the index calculating unit 104b adds a predetermined value to the index value of each subregion. On the other hand, when the region selecting unit 106 selects a subregion with a large index value as a region to be used for analysis and there is a subregion in which foreign matter is imaged, the index calculating unit 104b subtracts a predetermined value from the index value of each subregion. Here, the predetermined value is preferably a large value enough not to select the subregion in which the foreign matter is imaged as an observation region.

[Imaging process]

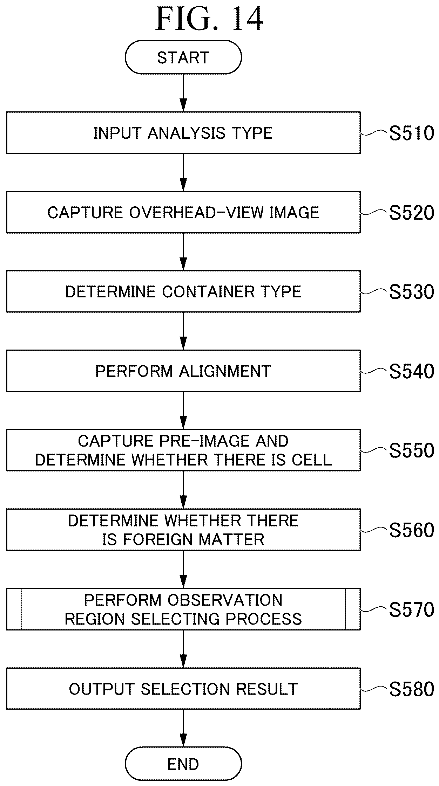

An imaging process that is performed by the observation device 1b will be described below with reference to FIGS. 14 to 16. The imaging process performed by the observation device 1b is different from the imaging process performed by the observation device 1 according to the first embodiment in the first preparation process.

FIG. 14 is a diagram illustrating an example of a flow of the first preparation process according to the present embodiment.

The processes of Steps S510 to S550 and Step S580 are the same as the processes of Steps S10 to S50 and Step S70 in FIG. 6, and thus a description thereof will be omitted.

Step S560: The foreign matter detecting unit 113b determines whether foreign matter is imaged in the pre-image. The foreign matter detecting unit 113b detects foreign matter, for example, on the basis of a result of image recognition of a subregion from the image determining unit 102. When foreign matter is detected, the foreign matter detecting unit 113b supplies foreign matter position information to the index calculating unit 104b. The foreign matter position information is information indicating a position of foreign matter in the pre-image.

Thereafter, the analysis device 10b performs the process of Step S570.

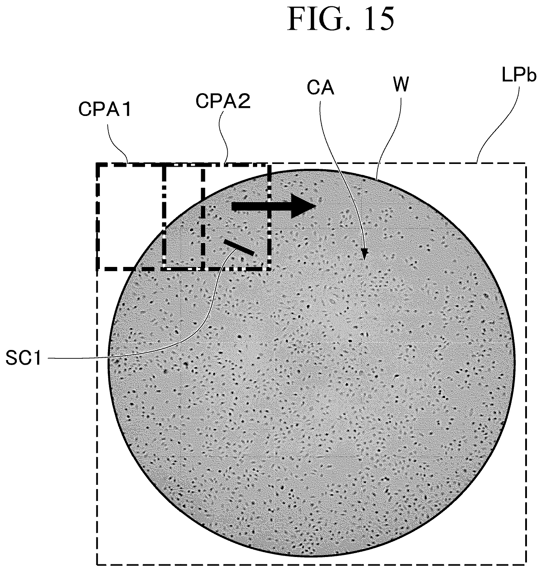

A case in which foreign matter is imaged in a pre-image will be described below with reference to FIG. 15. FIG. 15 is a diagram illustrating a pre-image in which foreign matter is imaged according to the present embodiment. In FIG. 15, a pre-image LPb is illustrated as an example of the pre-image acquired by the cell image acquiring unit 101. In the pre-image LPb, a scar SC1 is imaged in a subregion CPA2. The scar SC1 is an example of foreign matter.

The description of the first preparation process will be continued with reference back to FIG. 14.

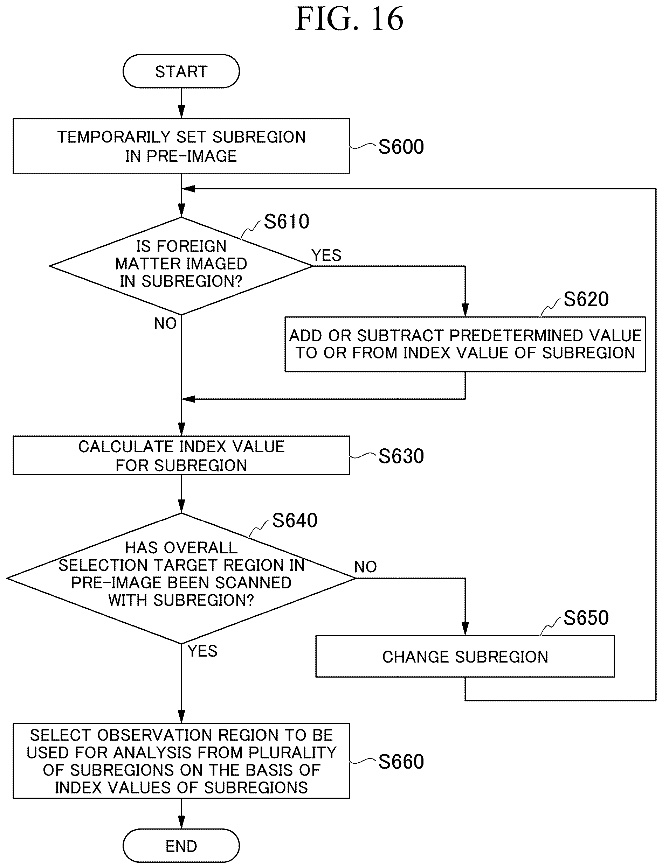

Step S570: The observation region selecting unit 103b performs an observation region selecting process. The observation region selecting process performed by the observation region selecting unit 103b is different from the observation region selecting process (FIG. 7) performed by the observation region selecting unit 103 according to the first embodiment in that the process flow illustrated in FIG. 16 is performed.

FIG. 16 is a diagram illustrating an example of a flow of the observation region selecting process according to the present embodiment.

The processes of Step S600 and Steps S640 to S660 are the same as the processes of Step S100 and Steps S120 to S140 in FIG. 7, and thus a description thereof will be omitted.

Step S610: The index calculating unit 104b determines whether foreign matter is imaged in a current subregion. The index calculating unit 104b determines whether foreign matter is imaged in a current subregion on the basis of the foreign matter position information supplied from the foreign matter detecting unit 113b.

When it is determined that foreign matter is imaged in a current subregion (Step S610: YES), the index calculating unit 104b performs the process of Step S620. On the other hand, when it is determined that foreign matter is not imaged in a current subregion (Step S610: NO), the index calculating unit 104b performs the process of Step S630.

Step S620: The index calculating unit 104b adds or subtracts a predetermined value to or from the index value of the subregion. When the region selecting unit 106 selects a subregion with the smallest index value as a region to be used for analysis, the index calculating unit 104b adds a predetermined value to the index value of the subregion. On the other hand, when the region selecting unit 106 selects a subregion with a largest index value as a region to be used for analysis, the index calculating unit 104b subtracts a predetermined value from the index value of the subregion.

Here, when the region selecting unit 106 selects a subregion with the smallest index value as a region to be used for analysis and a predetermined value is added to the index value of the subregion in which foreign matter is imaged, the index value of the subregion is large. Accordingly, the subregion is not selected as a region to be used for analysis.

Similarly, when the region selecting unit 106 selects a subregion with the largest index value as a region to be used for analysis and a predetermined value is subtracted from the index value of the subregion in which foreign matter is imaged, the index value of the subregion is small. Accordingly, the subregion is not selected as a region to be used for analysis.

Thereafter, the analysis device 10b performs the process of Step S630.

In the observation device 1b according to the present embodiment, since an index value can be appropriately calculated even when foreign matter is imaged in a pre-image, it is possible to exclude the region in which the foreign matter is imaged and to select a region to be used for analysis.

In the aforementioned embodiments, an example in which one well W is imaged in one pre-image and an observation region is determined for each well W has been described above, but the present invention is not limited thereto. An observation region for each well may be determined using a pre-image including a plurality of wells.

In this case, the microscope device 20 may acquire one pre-image by acquiring and connecting a plurality of images to generate one image. For example, images in which a plurality of neighboring wells in the well plate WP are imaged at a low magnification by the second optical system may be connected and used as one pre-image.

A plurality of (for example, four) wells in the well plate WP may be imaged once at a low magnification by the second optical system and used as a pre-image. The low magnification is a magnification lower than the magnification when one well W is imaged.

Accordingly, the image acquiring unit (the microscope device 20 in the embodiments) may acquire the second image (the pre-image in the embodiments) by acquiring and connecting a plurality of images to generate an image.

A part of the analysis device 10, 10a, or 10b according to the aforementioned embodiments, for example, the arithmetic operation unit 100, 100a, or 100b, may be realized by a computer. In this case, it may be realized by recording a program for realizing the aforementioned control function on a computer-readable recording medium and causing a computer system to read and execute the program recorded on the recording medium. The "computer system" mentioned herein is a computer system provided in the analysis device 10, 10a, or 10b and includes an OS or hardware such as peripherals. The "computer-readable recording medium" is a portable medium such as a flexible disk, a magneto-optical disc, a ROM, or a CD-ROM or a storage device such as a hard disk incorporated into a computer system. The "computer-readable recording medium" may include a medium that dynamically holds a program for a short time such as a communication line when the program is transmitted via a network such as the Internet or a communication line such as a telephone line or a medium that holds a program for a predetermined time such as a volatile memory in a computer system serving as a server or a client in that case. The program may be a program for realizing some of the aforementioned functions or may be a program for realizing the aforementioned functions in combination with another program stored in advance in the computer system.

A part or whole of the analysis device 10, 10a, or 10b according to the aforementioned embodiments may be realized by an integrated circuit such as a large scale integration (LSI) circuit. The functional blocks of the analysis device 10, 10a, or 10b may be individually integrated as processors, or some or all thereof may be integrated as a processor. The circuit integration is not limited to LSI, and may be realized using an exclusive circuit or a general-purpose processor. When circuit integration technology replacing LSI appears with advancement in semiconductor technology, an integrated circuit based on this technology may be used.

While embodiments of the present invention have been described above in detail with reference to the drawings, any specific configurations are not limited to these embodiments, and various design modifications or the like can be added without departing from the gist of the invention.

REFERENCE SIGNS LIST

1, 1a, 1b... Observation device
20... Microscope device
104, 104a, 104b... Index calculating unit
106... Region selecting unit

**Claims**

1. An observation device that analyzes a cell image, the observation device comprising:

an image acquiring unit configured to acquire a second image in a region wider than a region used for analysis at a pixel resolution lower than a pixel resolution of a first image used for the analysis;
an index calculating unit configured to calculate a value of an index for a cell in each of subregions included in the second image and corresponding to the region used for the analysis; and
a region selecting unit configured to select a region to be used for the analysis from the subregions on the basis of the value of the index calculated by the index calculating unit.

2. The observation device according to claim 1, wherein the index includes a first index and a second index.

3. The observation device according to claim 2, wherein the first index and the second index are selected from indices (A) to (H): (A) a cell-covered area ratio; (B) a cell density distribution; (C) a cell number; (D) a cell fluorescence intensity; (E) a cell crowdedness; (F) a cell circularity; (G) a one-cell area; and (H) a cell multinuclear factor.

4. The observation device according to any one of claims 1 to 3, further comprising a first input unit configured to receive an input of an analysis type,
wherein the index calculating unit determines the index of which a value is to be calculated according to the analysis type.

5. The observation device according to any one of claims 1 to 4, further comprising a second input unit configured to receive an input of a cell type,
wherein the index calculating unit determines the index of which a value is to be calculated according to the cell type.

6. The observation device according to any one of claims 1 to 5, further comprising a third input unit configured to receive an input of a determination method for the region to be used for the analysis,
wherein the determination method includes a method of determining a region to be used for the analysis using the index calculating unit and the region selecting unit and a method of determining a predetermined region in the second image as the region to be used for the analysis.

7. The observation device according to any one of claims 1 to 6, wherein the second image is an image obtained by imaging cells disposed in a plurality of storage portions included in a container for each of the storage portions.

8. The observation device according to claim 7, further comprising an image determining unit configured to determine whether there is a cell in the second image,
wherein the index calculating unit calculates the value of the index in the second image in which there is a cell.

9. The observation device according to any one of claims 1 to 8, wherein the image acquiring unit includes a detection optical system including a plurality of objective lenses,

wherein the first image is acquired using a first objective lens out of the plurality of objective lenses, and
wherein the second image is acquired using a second objective lens with a magnification lower than that of the first objective lens out of the plurality of objective lenses.

10. The observation device according to any one of claims 1 to 9, wherein the image acquiring unit includes a first optical system for acquiring the first image and a second optical system for acquiring the second image.

11. The observation device according to any one of claims 1 to 10, wherein the image acquiring unit acquires the second image by acquiring and connecting a plurality of images to generate one image.

12. The observation device according to any one of claims 1 to 11, wherein the index calculating unit adds a predetermined value to the value of the index in each subregion when the region selecting unit selects the subregion with a small value of the index as the region to be used for the analysis and there is a subregion in which foreign matter is imaged, and
wherein the index calculating unit subtracts a predetermined value from the value of the index in each subregion when the region selecting unit selects the subregion with a large value of the index as the region to be used for the analysis and there is the subregion in which foreign matter is imaged.

13. The observation device according to any one of claims 1 to 12, wherein the region selecting unit selects a plurality of regions as a candidate for the region to be used for the analysis out of the plurality of subregions on the basis of the index calculated by the index calculating unit.

14. An observation method of analyzing a cell image, the observation method comprising:

acquiring a second image in a region wider than a region used for analysis at a pixel resolution lower than a pixel resolution of a first image used for the analysis;

calculating a value of an index for a cell in each of subregions included in the second image and corresponding to the region used for the analysis; and

selecting a region to be used for the analysis from the subregions on the basis of the value of the index calculated in the calculating of a value of an index.

15. A program causing a computer that analyzes a cell image to perform:

acquiring a second image in a region wider than a region used for analysis at a pixel resolution lower than a pixel resolution of a first image used for the analysis;

calculating a value of an index for a cell in each of subregions included in the second image and corresponding to the region used for the analysis; and

selecting a region to be used for the analysis from the subregions on the basis of the value of the index calculated in the calculating of a value of an index.

# FIG. 1

EP 4 763 968 A1

# FIG. 2

**1**

**20** MICROSCOPE DEVICE

**24** OBJECTIVE LENS DRIVE UNIT

**22** IMAGING UNIT

**23**

**WP**

**21**

**25** MOTOR-DRIVEN STAGE DRIVE UNIT

**10** ANALYSIS DEVICE

**500** CONTROL SIGNAL OUTPUT UNIT

**100** ARITHMETIC OPERATION UNIT

**101** CELL IMAGE ACQUIRING UNIT

**102** IMAGE DETERMINING UNIT

**103** OBSERVATION REGION SELECTING UNIT

**104** INDEX CALCULATING UNIIT

**105** INDEX CALCULATION REGION CHANGING UNIT

**106** REGION SELECTING UNIIT

**107** ANALYSIS UNIT

**108** AUTO-FOCUSING UNIT

**110** ABNORMALITY DETECTING UNIT

**111** ILLUMINATION CONDITION DETERMINING UNIT

**115** ANALYSIS TYPE INPUT UNIT

**200** STORAGE UNIT

**A1** INDEX INFORMATION

**30** DISPLAY UNIT

**300** RESULT OUTPUT UNIT

**40** OPERATION UNIT

**400** OPERATION DETECTING UNIT

EP 4 763 968 A1

# FIG. 3

EP 4 763 968 A1

# FIG. 4

| | INDEX | DEFINITION |
|---|---|---|
| 1 | CELL-COVERED AREA RATIO | "RATIO OF AREA OF CELLS INCLUDED PREDETERMINED REGION TO TOTAL AREA OF PREDETERMINED REGION" IN PREDETERMINED REGION |
| 2 | CELL DENSITY DISTRIBUTION | STANDARD DEVIATION BASED ON NUMBER DISTRIBUTION OF CELLS INCLUDED IN PREDETERMINED REGION |
| 3 | CELL NUMBER | NUMBER OF CELLS INCLUDED IN PREDETERMINED REGION |
| 4 | CELL FLUORESCENCE INTENSITY | AVERAGE VALUE OF FLUORESCENT LUMINANCE VALUES OF CELLS INCLUDED IN PREDETERMINED REGION |
| 5 | CELL CROWDEDNESS | NUMBER OF CELLS IN WHICH DISTANCE OF EACH CELL INCLUDED IN PREDETERMINED REGION TO NEAREST CELL IS EQUAL TO OR LESS THAN PREDETERMINED DISTANCE |
| 6 | CELL CIRCULARITY | NUMBER OF CELLS IN WHICH VALUE OF "$4\pi$ (AREA) (CIRCUMFERENCE)" OF EACH CELL INCLUDED IN PREDETERMINED REGION IS EQUAL TO OR LESS THAN PREDETERMINED VALUE |
| 7 | AREA PER CELL | AVERAGE VALUE OF AREAS OF CELLS INCLUDED IN PREDETERMINED REGION |
| 8 | CELL MULTINUCLEAR FACTOR | AVERAGE VALUE OF NUMBER OF NUCLEI OF CELLS INCLUDED IN PREDETERMINED REGION |

# FIG. 5

| # | FIRST INDEX | STATISTIC | SECOND INDEX | STATISTIC |
|---|---|---|---|---|
| 1 | CELL-COVERED AREA RATIO | REPRESENTATIVE VALUE | CELL DENSITY DISTRIBUTION | MINIMUM VALUE |
| 2 | CELL CIRCULARITY | REPRESENTATIVE VALUE | ONE-CELL AREA | REPRESENTATIVE VALUE |
| 3 | CELL FLUORESCENCE INTENSITY | MAXIMUM VALUE | CELL NUMBER | MAXIMUM VALUE |
| 4 | CELL CROWDEDNESS | REPRESENTATIVE VALUE | CELL NUMBER | REPRESENTATIVE VALUE |
| 5 | CELL MULTINUCLEAR FACTOR | MINIMUM VALUE | ONE-CELL AREA | REPRESENTATIVE VALUE |
| 6 | CELL FLUORESCENCE INTENSITY | MINIMUM VALUE | CELL NUMBER | MAXIMUM VALUE |
| 7 | AREA PER CELL | 75% VALUE | CELL NUMBER | MAXIMUM VALUE |
| 8 | MAXIMUM CELL CIRCULARITY | MAXIMUM VALUE | | |

# FIG. 6

START

INPUT ANALYSIS TYPE — S10

CAPTURE OVERHEAD-VIEW IMAGE — S20

DETERMINE CONTAINER TYPE — S30

PERFORM ALIGNMENT — S40

CAPTURE PRE-IMAGE AND
DETERMINE WHETHER THERE IS CELL — S50

PERFORM OBSERVATION
REGION SELECTING PROCESS — S60

OUTPUT SELECTION RESULT — S70

END

# FIG. 7

START

TEMPORARILY SET SUBREGION
IN PRE-IMAGE — S100

CALCULATE INDEX VALUE FOR SUBREGION — S110

S120
HAS OVERALL
SELECTION TARGET REGION
IN PRE-IMAGE BEEN SCANNED
WITH SUBREGION?

NO

S130
CHANGE SUBREGION

YES

SELECT OBSERVATION REGION TO BE
USED FOR ANALYSIS FROM PLURALITY
OF SUBREGIONS ON THE BASIS
OF INDEX VALUES OF SUBREGIONS — S140

END

FIG. 8

# FIG. 9

START

DETERMINE MATERIAL OF CONTAINER — S210

PERFORM AUTO-FOCUSING — S220

CAPTURE LIGHT-FIELD IMAGE AND
FLUORESCENT IMAGE AS PRE-ROI IMAGE — S230

DETECT ABNORMALITY IN CELL SHAPE AND
ABNORMALITY IN DEW CONDENSATION ON THE BASIS
OF LIGHT-FIELD IMAGE, DETECT ABNORMALITY IN
SIGNAL ON THE BASIS OF FLUORESCENT IMAGE,
AND DETERMINE ILLUMINATION CONDITIONS — S240

OUTPUT DETECTION RESULT — S250

END

# FIG. 10

START

↓

CAPTURE BRIGHT-FIELD IMAGE AND
FLUORESCENT IMAGE AS MAIN IMAGE — S310

↓

ANALYZE MAIN IMAGE — S320

↓

OUTPUT ANALYSIS RESULT — S330

↓

END

## FIG. 11

_1a_

**ANALYSIS DEVICE** ~10a

**CONTROL SIGNAL OUTPUT UNIT** ~500

**MICROSCOPE DEVICE** ~20

**ARITHMETIC OPERATION UNIT** ~100a

**CELL IMAGE ACQUIRING UNIT** ~101

**IMAGE DETERMINING UNIT** ~102

**STORAGE UNIT** ~200

**INDEX INFORMATION** ~A1a

**IMAGING UNIT** ~22

**OBJECTIVE LENS DRIVE UNIT** 24

~23

**AUTO-FOCUSING UNIT** ~108

**OBSERVATION REGION SELECTING UNIT** ~103a

**INDEX CALCULATING UNIIT** ~104a

WP

**INDEX CALCULATION REGION CHANGING UNIT** ~105

**ABNORMALITY DETECTING UNIT** ~110

**MOTOR-DRIVEN STAGE DRIVE UNIT** ~25

21

**REGION SELECTING UNIIT** ~106

**ILLUMINATION CONDITION DETERMINING UNIT** ~111

**CELL TYPE INPUT UNIT** ~112a

107~ **ANALYSIS UNIT**

30~ **DISPLAY UNIT**

**RESULT OUTPUT UNIT** ~300

40~ **OPERATION UNIT**

**OPERATION DETECTING UNIT** ~400

EP 4 763 968 A1

# FIG. 12

START

TEMPORARILY SET SUBREGION
IN PRE-IMAGE — S400

CALCULATE INDEX VALUE FOR SUBREGION — S410

S420
HAS OVERALL
SELECTION TARGET REGION
IN PRE-IMAGE BEEN SCANNED
WITH SUBREGION?

NO → CHANGE SUBREGION — S430

YES

SELECT OBSERVATION REGION TO BE
USED FOR ANALYSIS FROM PLURALITY
OF SUBREGIONS ON THE BASIS — S440
OF INDEX VALUES OF SUBREGIONS

END

# FIG. 13

EP 4 763 968 A1

# FIG. 14

START

INPUT ANALYSIS TYPE — S510

CAPTURE OVERHEAD-VIEW IMAGE — S520

DETERMINE CONTAINER TYPE — S530

PERFORM ALIGNMENT — S540

CAPTURE PRE-IMAGE AND DETERMINE WHETHER THERE IS CELL — S550

DETERMINE WHETHER THERE IS FOREIGN MATTER — S560

PERFORM OBSERVATION REGION SELECTING PROCESS — S570

OUTPUT SELECTION RESULT — S580

END

FIG. 15

# FIG. 16

START

TEMPORARILY SET SUBREGION
IN PRE-IMAGE — S600

IS FOREIGN
MATTER IMAGED
IN SUBREGION? — S610

YES →

ADD OR SUBTRACT PREDETERMINED VALUE
TO OR FROM INDEX VALUE OF SUBREGION — S620

NO

CALCULATE INDEX VALUE
FOR SUBREGION — S630

HAS OVERALL
SELECTION TARGET REGION IN
PRE-IMAGE BEEN SCANNED
WITH SUBREGION? — S640

NO →

CHANGE SUBREGION — S650

YES

SELECT OBSERVATION REGION TO BE
USED FOR ANALYSIS FROM PLURALITY
OF SUBREGIONS ON THE BASIS OF
INDEX VALUES OF SUBREGIONS — S660

END

## INTERNATIONAL SEARCH REPORT

| International application No. |
| --- |
| **PCT/JP2024/023605** |

| A. | CLASSIFICATION OF SUBJECT MATTER |
| --- | --- |

*C12M 1/34*(2006.01)i; *C12M 1/00*(2006.01)i; *C12Q 1/02*(2006.01)i; *G02B 21/00*(2006.01)i
FI:   C12M1/34 D; C12M1/00 A; C12Q1/02; G02B21/00

According to International Patent Classification (IPC) or to both national classification and IPC

| B. | FIELDS SEARCHED |
| --- | --- |

Minimum documentation searched (classification system followed by classification symbols)

C12M1/34; C12M1/00; C12Q1/02; G02B21/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT | |
| --- | --- | --- |
| Category* | Citation of document, with indication, where appropriate, of the relevant passages | Relevant to claim No. |
| X | JP 2018-157830 A (FUJIFILM CORPORATION) 11 October 2018 (2018-10-11) claims, paragraphs [0003], [0026], [0037]-[0055], [0067], [0076], [0087], drawings (Family: none) | 1-15 |

| ☐ Further documents are listed in the continuation of Box C. | ☐ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **07 August 2024** | **20 August 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023132533 A

- US 9280693 B